# EUROPEAN PATENT APPLICATION

(11) **EP 4 834 918 A2**
(43) Date of publication of application: **04.11.2026**
(21) Application number: 26203006.7
(22) Date of filing: 11.02.2020
(51) Int. Cl.: C07K 14/435

(54) **MODULATION OF WNT SIGNALLING IN OCCULAR DISORDERS**

(30) Priority: 11.02.2019 US 201962803835 P
(62) Document number(s) of the earlier application(s) in accordance with Art. 76 EPC: 20755490.8 / 3 924 381
(71) Applicant: Surrozen Operating, Inc., South San Francisco, CA 94080 (US)
(72) Inventor: LI, Yang, Mountain View, CA 94040 (US); TU, Shengjiang, Foster City, CA 94404 (US); LEE, Sungjin, Palo Alto, CA 94304 (US); YEH, Wen-Chen, Belmont, CA 94002 (US)
(74) Representative: Cooley (UK) LLP

(57) **Abstract**

The present invention provides methods of treating ocular disorders with modulators of the WNT signaling pathway. In particular the ocular disorders are retinopathies. Also provided are methods of dosing and pharmaceutical compositions.

## Description

### CROSS REFERENCE TO RELATED APPLICATIONS

This application claims priority to U.S. Provisional Application No. 62/803,835, filed February 11, 2019, which is incorporated by reference herein in its entirety.

### STATEMENT REGARDING SEQUENCE LISTING

The Sequence Listing associated with this application is provided in text format in lieu of a paper copy, and is hereby incorporated by reference into the specification. The name of the text file containing the Sequence Listing is SRZN_013_01WO_ST25.txt. The text file is about 27 KB, created on February 10, 2020, and is being submitted electronically via EFS-Web.

### FIELD OF THE INVENTION

The present invention provides WNT signal modulators to treat various ocular disorders. In particular, provided are treatments for vascular diseases of the eye, also known as retinopathies.

### BACKGROUND OF THE INVENTION

The vertebral retina is a thin layer of nerve tissue in the back of the eye. It is responsible for detecting visual stimuli and is the first station for visual information processing. For its proper function, the retinal vasculature is an indispensable source of nutrients and oxygen. The retina is metabolically highly active. Due to the photoreceptors which consume the vast amount of oxygen, a gram of retina shows the highest oxygen consumption rate than any other organs in body. To serve as an effective nutrients and oxygen, the retinal vasculature is positioning in retina as a stereotyped architecture consisting of three planal vascular plexuses on one side and the choriocapillaries on the other. The inner vascularization initially begins on vitreal surface of retina, giving rise to a primary vascular plexus. After the superficial radial expansion of the vascular plexus, vertical penetration of vessels into retina forms two additional intraretinal capillary plexuses at inner plexiform layer (IPL) and outer plexiform layer (OPL). Due to the functional and structural relationship between blood vessels and retina, the aberrant vessel development or the vascular damages are directly linked to the function of retina, which causes various types of retinopathy and degeneration.

WNT signaling has been implicated as an important pathway for the vascular development in retina. Growing genetic evidences from human and rodent studies further support the importance of WNT signaling in retinal vasculature (Wang et al., 2018, Prog Retin Eye Res. 2018 Dec 1. pii: S1350-9462(18)30046-6). Human mutations in genes encoding either receptors (Fzd4, Lrp5, Tspan12) or a ligand (norrin) involved in the WNT signaling result in a variety of inherited vitreoretinopathies. The individual genetic mutant mouse of the genes (Fzd4, Lrp5, Tspan12, norrin) has also shown the typical phenotypes of aberrant vasculature seen in human retinopathy. This not only allowed better understanding of the retinopathy disease progression, but also opened the possibility of retinopathy treatment through WNT signal modulation.

Retinopathy, in particular, diabetic retinopathy, can be divided into early and late stages. In the early stages, also known as non-proliferative retinopathy, there may be a slight deterioration in the small blood vessels of the retina, portions of the vessels may swell and leak fluid into the surrounding retinal tissue. Late stage retinopathy involves significant neovascularization as well as microaneurysms and hemorrhages in the retinal area (see, e.g., Grading Diabetic Retinopathy from Stereoscopic Color Fundus Photographs-An Extension of the Modified Airlie House Classification. (1991) Ophthalmology, 98(5), 786-806*).*

Familial Exudative Vitreoretinopathy (FEVR) is the genetic eye disease with poor formation of intraocular vasculature. Over 50% of FEVR patients show mutations in one of the genes encoding Fzd4, Lrp5, Tspan12, or norrin. Norrin, WNT signal ligand, transmits a signal to the endothelial cells through a receptor complex composed of Fzd4/Lrp5/Tspan12 for normal retinal vascularization in eye. However, in FEVR patients, the mutations in genes encoding the one of norrin, Fzd4, Lrp5, or/and Tspan12 cause the immature vascular development in retina. The resulting formation of the avascular region creates a retinal ischemic area, which is primary damage to the retina. The ischemic condition induces the production of vascular endothelial growth factor (VEGF) and angiopoietin2 (Ang2), leading to neovascularization and vascular tuft formation. The newly generated abnormal blood vessels formed can be easily broken, leading to the secondary damage of retina due to exudation and hemorrhage. Disease progression of diabetic retinopathy (DR) is also similar to that of FEVR or other genetic vascular malformation or insufficiency diseases. Hyperglycemia induces retinal vessel damage, leading to vaso-obliteration, ischemia, neovascularization, and hemorrhage, eventually leading the retinopathy.

While genetic data has suggested importance of WNT signaling in establishing the proper vascular structure in the eye, whether activation of WNT signaling post-developmentally would lead to improvement in vascular structure is unknown. Certain reports have even suggested that antagonizing rather than agonizing WNT signaling would be beneficial in retinopathy. Therefore, understanding the retinopathy disease progression and the WNT signal involvement extends to the possibilities of new treatments. For the proper treatment of retinopathy, a need exists to control WNT agonist and antagonist signaling depending on the disease stage. The present invention provides methods to control WNT signaling agonism and antagonism in different stage of disease development of retinopathy.

### SUMMARY OF THE INVENTION

The present invention is based, in part, upon the use of WNT signaling agonists and antagonists to regulate aberrant vascular formation in retinopathy indication.

The present invention provides a method of treating a subject suffering from the retinopathy comprising administering the subject, an engineered WNT signaling modulator. In certain embodiments, the WNT signaling modulator is an engineered WNT agonist or an engineered WNT antagonist. In further embodiments the engineered WNT agonist and WNT antagonist comprise binding compositions that bind to one or more Fzd receptors and binding compositions that bind to one or more LRP receptors or Tspan12 receptors. In further embodiments, the binding compositions of the engineered WNT agonist are selected from the group consisting of a Fzd4 binding composition, a Lrp5 binding composition, a Lrp6 binding composition, a LRP5/6 binding composition, and a Tspan12 binding composition.

In some embodiments, the engineered WNT agonist or WNT antagonist are administered independently at early and/or late stages of retinopathy. In alternative embodiments, the WNT agonist and WNT antagonist are administered sequentially at early and/or late stages of retinopathy, or the WNT agonist and WNT antagonist are co-administered at early and/or late stages of retinopathy. In further embodiments, the WNT agonist is administered before or after the WNT antagonist.

In some embodiments, the WNT agonist and/or the WNT antagonists is administered with a binding composition specific for either VEGF and/or Ang2. In certain embodiments, the binding composition specific for VEGF or Ang2 is an antagonist of VEGF or Ang2 activity. In further embodiments, the VEGF antagonist is selected from the group consisting of: bevacizumab, ranibizumab, aflibercept, ramucirumab, and tanibirumab. In other embodiments, the Ang2 antagonist is selected from the group consisting of nesvacumab, AMG780, and MEDI3617.

In certain embodiments, the retinopathy is a retinal vascular disease. In the some embodiments, the retinal vascular disease is caused by inhibition of vascular development. In alternate embodiments, the retinal vascular disease is caused by excessive angiogenesis. In particular embodiments, the retinal vascular disease is selected from the group consisting of: familiar exudative vitreoretionopathy (FEVR), exudative vitreoretinopathy, Norrie disease, diabetic retinopathy (DR), age-related macular degeneration (AMD), retinopathy of prematurity (ROP), osteoporosis-psuedoglioma syndrome (OPPG), retinal vein occlusion, and Coats disease.

### DESCRIPTION OF THE DRAWINGS

Figs. 1A and 1B provide a description of the WNT surrogate molecules used. Fig. 1A shows a graphical representation of the WNT surrogate molecules, and Fig. 1B provides the clone names and sequence identifiers for each component of the WNT surrogate molecules.
Figs. 2A-2H are graphs showing WNT signaling activity, as measured by the SuperTop Flash (STF) assay, in cells treated with the indicated WNT surrogate molecules and RSPO. Figs. 2A-2D show little to no WNT signaling activity in untransfected HEK293 cells treated with various mono-FZD4 WNT surrogates and 20 nM RSPO. In contrast, Figs. 2E-2H show HEK293 cells transfected with the human FZD4 gene having WNT signaling activity when treated with various FZD4 WNT surrogates and 20 nM RSPO.
Fig. 3 shows semi-quantitative PCR analysis of FZD4 over-expressing HEK293 cells.
Figs. 4A-4P show WNT signaling activity (Figs. 4A-4D) and Axin2 expression (Figs. 4E-4H) in bEnd.3 cells (mouse brain endothelial cell line used in vascular studies) containing a luciferase gene controlled by a WNT-responsive promoter; or WNT signaling activity (Figs. 4I-4L) and Axin2 expression (Figs. 4M-4P) in HRMEC (Primary Human Retinal Microvascular Endothelial Cells) containing a luciferase gene controlled by a WNT-responsive promoter.
Figs. 5A-5B show semi-quantitative PCR analysis of various WNT receptor gene expression in bEnd.3 cells (Fig. 5A) and HRMEC (Fig. 5B).
Figs. 6A-6F show the effect of treatment with various FZD4 WNT surrogates with or without added RSPO on WNT signaling activity in bEnd.3 cells (Figs. 6A-6C) or HRMEC cells (Figs. 6D-6F).
Figs. 7A-7B show the experimental design using various FZD4 WNT surrogate molecules in a rat model of oxygen-induced retinopathy model. Fig. 7A shows a timeline of the oxygen-induced retinopathy model; and Fig. 7B provides details on arms of the study.
Figs. 8A-8B shows retinal vascular growth and pathological pre-retinal neovascularization following treating with either anti-VEGF or FZD4 WNT surrogate molecules. Fig. 8A shows fluorescent staining of rat retinal flatmounts. Fig. 8B shows quantitative analysis by computer assisted image analysis of vascular growth and neovascularization.

### DETAILED DESCRIPTION

As used herein, including the appended claims, the singular forms of words such as "a," "an," and "the," include their corresponding plural references unless the context clearly dictates otherwise.

All references cited herein are incorporated by reference to the same extent as if each individual publication, patent application, or patent, was specifically and individually indicated to be incorporated by reference.

### I. Definitions.

"Activity" of a molecule may describe or refer to the binding of the molecule to a ligand or to a receptor, to catalytic activity, to the ability to stimulate gene expression, to antigenic activity, to the modulation of activities of other molecules, and the like. "Activity" of a molecule may also refer to activity in modulating or maintaining cell-to-cell interactions, e.g., adhesion, or activity in maintaining a structure of a cell, e.g., cell membranes or cytoskeleton. "Activity" may also mean specific activity, e.g., [catalytic activity]/[mg protein], or [immunological activity]/[mg protein], or the like.

The terms "administering" or "introducing" or "providing", as used herein, refer to delivery of a composition to a cell, to cells, tissues and/or organs of a subject, or to a subject. Such administering or introducing may take place in vivo, in vitro or ex vivo.

As used herein, the term "antibody" means an isolated or recombinant binding agent that comprises the necessary variable region sequences to specifically bind an antigenic epitope. Therefore, an antibody is any form of antibody or fragment thereof that exhibits the desired biological activity, e.g., binding the specific target antigen. Thus, it is used in the broadest sense and specifically covers monoclonal antibodies (including full-length monoclonal antibodies), polyclonal antibodies, human antibodies, humanized antibodies, chimeric antibodies, nanobodies, diabodies, multispecific antibodies (e.g., bispecific antibodies), and antibody fragments including but not limited to scFv, Fab, and Fab2, so long as they exhibit the desired biological activity.

"Antibody fragments" comprise a portion of an intact antibody, for example, the antigen-binding or variable region of the intact antibody. Examples of antibody fragments include Fab, Fab', F(ab')2, and Fv fragments; diabodies; linear antibodies (e.g., Zapata et al., Protein Eng. 8(10): 1057-1062 (1995)); single-chain antibody molecules (e.g., scFv); and multispecific antibodies formed from antibody fragments. Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an F(ab')2 fragment that has two antigen combining sites and is still capable of cross-linking antigen.

The term "antigen" refers to a molecule or a portion of a molecule capable of being bound by a selective binding agent, such as an antibody, and 30 additionally capable of being used in an animal to produce antibodies capable of binding to an epitope of that antigen. In certain embodiments, a binding agent (e.g., a WNT surrogate molecule or binding region thereof, or a WNT antagonist) is said to specifically bind an antigen when it preferentially recognizes its target antigen in a complex mixture of proteins and/or macromolecules.

The term "antigen-binding fragment" as used herein refers to a polypeptide fragment that contains at least one CDR of an immunoglobulin heavy and/or light chain, or of a VHH/sdAb (single domain antibody) or Nanobody^{®} (Nab), that binds to the antigen of interest, in particular to one or more Fzd receptors, or to LRP5 and/or LRP6. In this regard, an antigen-binding fragment of the herein described antibodies may comprise 1, 2, 3, 4, 5, or all 6 CDRs of a VH and VL from antibodies that bind one or more Fzd receptors or LRP5 and/or LRP6.

As used herein, the terms "biological activity" and "biologically active" refer to the activity attributed to a particular biological element in a cell. For example, the "biological activity" of a WNT agonist, or fragment or variant thereof refers to the ability to mimic or enhance WNT signals. As another example, the biological activity of a polypeptide or functional fragment or variant thereof refers to the ability of the polypeptide or functional fragment or variant thereof to carry out its native functions of, e.g., binding, enzymatic activity, etc. As a third example, the biological activity of a gene regulatory element, e.g. promoter, enhancer, Kozak sequence, and the like, refers to the ability of the regulatory element or functional fragment or variant thereof to regulate, i.e. promote, enhance, or activate the translation of, respectively, the expression of the gene to which it is operably linked.

The term "bifunctional antibody," as used herein, refers to an antibody that comprises a first arm having a specificity for one antigenic site and a second arm having a specificity for a different antigenic site, i.e., the bifunctional antibodies have a dual specificity.

"Bispecific antibody" is used herein to refer to a full-length antibody that is generated by quadroma technology (see Milstein et al., Nature, 305(5934): 537-540 (1983)), by chemical conjugation of two different monoclonal antibodies (see, Staerz et al., Nature, 314(6012): 628-631 (1985)), or by knob-into-hole or similar approaches, which introduce mutations in the Fc region (see Holliger et al., Proc. Natl. Acad. Sci. USA, 90(14): 6444-6448 (1993)), resulting in multiple different immunoglobulin species of which only one is the functional bispecific antibody. A bispecific antibody binds one antigen (or epitope) on one of its two binding arms (one pair of HC/LC), and binds a different antigen (or epitope) on its second arm (a different pair of HC/LC). By this definition, a bispecific antibody has two distinct antigen-binding arms (in both specificity and CDR sequences), and is monovalent for each antigen to which it binds.

By "comprising," it is meant that the recited elements are required in, for example, the composition, method, kit, etc., but other elements may be included to form the, for example, composition, method, kit etc. within the scope of the claim. For example, an expression cassette "comprising" a gene encoding a therapeutic polypeptide operably linked to a promoter is an expression cassette that may include other elements in addition to the gene and promoter, e.g. poly-adenylation sequence, enhancer elements, other genes, linker domains, etc.

By "consisting essentially of," it is meant a limitation of the scope of the, for example, composition, method, kit, etc., described to the specified materials or steps that do not materially affect the basic and novel characteristic(s) of the, for example, composition, method, kit, etc. For example, an expression cassette "consisting essentially of" a gene encoding a therapeutic polypeptide operably linked to a promoter and a polyadenylation sequence may include additional sequences, e.g. linker sequences, so long as they do not materially affect the transcription or translation of the gene. As another example, a variant, or mutant, polypeptide fragment "consisting essentially of" a recited sequence has the amino acid sequence of the recited sequence plus or minus about 10 amino acid residues at the boundaries of the sequence based upon the full length naive polypeptide from which it was derived, e.g. 10, 9, 8, 7, 6, 5, 4, 3, 2 or 1 residue less than the recited bounding amino acid residue, or 1, 2, 3, 4, 5, 6, 7, 8, 9, or 10 residues more than the recited bounding amino acid residue.

By "consisting of," it is meant the exclusion from the composition, method, or kit of any element, step, or ingredient not specified in the claim. For example, a polypeptide or polypeptide domain "consisting of" a recited sequence contains only the recited sequence.

A "control element" or "control sequence" is a nucleotide sequence involved in an interaction of molecules that contributes to the functional regulation of a polynucleotide, including replication, duplication, transcription, splicing, translation, or degradation of the polynucleotide. The regulation may affect the frequency, speed, or specificity of the process, and may be enhancing or inhibitory in nature. Control elements known in the art include, for example, transcriptional regulatory sequences such as promoters and enhancers. A promoter is a DNA region capable under certain conditions of binding RNA polymerase and initiating transcription of a coding region usually located downstream (in the 3' direction) from the promoter.

An "expression vector" is a vector, e.g. plasmid, minicircle, viral vector, liposome, and the like as discussed herein or as known in the art, comprising a region which encodes a gene product of interest, and is used for effecting the expression of the gene product in an intended target cell. An expression vector also comprises control elements, e.g. promoters, enhancers, UTRs, miRNA targeting sequences, etc., operatively linked to the encoding region to facilitate expression of the gene product in the target. The combination of control elements and a gene or genes to which they are operably linked for expression is sometimes referred to as an "expression cassette," a large number of which are known and available in the art or can be readily constructed from components that are available in the art.

As used herein, the term "FR set" refers to the four flanking amino acid sequences which frame the CDRs of a CDR set of a heavy or light chain V region. Some FR residues may contact bound antigen; however, FRs are primarily responsible for folding the V region into the antigen-binding site, particularly the FR residues directly adjacent to the CDRs. Within FRs, certain amino residues and certain structural features are very highly conserved. In this regard, all V region sequences contain an internal disulfide loop of around 90 amino acid residues. When the V regions fold into a binding-site, the CDRs are displayed as projecting loop motifs which form an antigen-binding surface. It is generally recognized that there are conserved structural regions of FRs which influence the folded shape of the CDR loops into certain "canonical" structures-regardless of the precise CDR amino acid sequence. Further, certain FR residues are known to participate in non-covalent interdomain contacts which stabilize the interaction of the antibody heavy and light chains.

The terms "individual," "host," "subject," and "patient" are used interchangeably herein, and refer to a mammal, including, but not limited to, human and non-human primates, including simians and humans; mammalian sport animals (e.g., horses); mammalian farm animals (e.g., sheep, goats, etc.); mammalian pets (dogs, cats, etc.); and rodents (e.g., mice, rats, etc.).

A "monoclonal antibody" refers to a homogeneous antibody population wherein the monoclonal antibody is comprised of amino acids (naturally occurring and non-naturally occurring) that are involved in the selective binding of an epitope. Monoclonal antibodies are highly specific, being directed against a single epitope. The term "monoclonal antibody" encompasses not only intact monoclonal antibodies and full-length monoclonal antibodies, but also fragments thereof (such as Fab, Fab', F(ab')2, Fv), single chain (scFv), Nanobodies^{®}, variants thereof, fusion proteins comprising an antigen-binding fragment of a monoclonal antibody, humanized monoclonal antibodies, chimeric monoclonal antibodies, and any other modified configuration of the immunoglobulin molecule that comprises an antigen- binding fragment (epitope recognition site) of the required specificity and the ability to bind to an epitope, including WNT surrogate molecules disclosed herein. It is not intended to be limited as regards the source of the antibody or the manner in which it is made (e.g., by hybridoma, phage selection, recombinant expression, transgenic animals, etc.). The term includes whole immunoglobulins as well as the fragments etc. described above under the definition of "antibody".

The term "native" or "wild-type" as used herein refers to a nucleotide sequence, e.g. gene, or gene product, e.g. RNA or protein, that is present in a wild-type cell, tissue, organ or organism. The term "variant" as used herein refers to a mutant of a reference polynucleotide or polypeptide sequence, for example a native polynucleotide or polypeptide sequence, i.e. having less than 100% sequence identity with the reference polynucleotide or polypeptide sequence. Put another way, a variant comprises at least one amino acid difference (e.g., amino acid substitution, amino acid insertion, amino acid deletion) relative to a reference polynucleotide sequence, e.g. a native polynucleotide or polypeptide sequence. For example, a variant may be a polynucleotide having a sequence identity of 50% or more, 60% or more, or 70% or more with a full length native polynucleotide sequence, e.g. an identity of 75% or 80% or more, such as 85%, 90%, or 95% or more, for example, 98% or 99% identity with the full length native polynucleotide sequence. As another example, a variant may be a polypeptide having a sequence identity of 70% or more with a full length native polypeptide sequence, e.g. an identity of 75% or 80% or more, such as 85%, 90%, or 95% or more, for example, 98% or 99% identity with the full length native polypeptide sequence. Variants may also include variant fragments of a reference, e.g. native, sequence sharing a sequence identity of 70% or more with a fragment of the reference, e.g. native, sequence, e.g. an identity of 75% or 80% or more, such as 85%, 90%, or 95% or more, for example, 98% or 99% identity with the native sequence.

"Operatively linked" or "operably linked" refers to a juxtaposition of genetic elements, wherein the elements are in a relationship permitting them to operate in the expected manner. For instance, a promoter is operatively linked to a coding region if the promoter helps initiate transcription of the coding sequence. There may be intervening residues between the promoter and coding region so long as this functional relationship is maintained.

As used herein, the terms "polypeptide," "peptide," and "protein" refer to polymers of amino acids of any length. The terms also encompass an amino acid polymer that has been modified; for example, to include disulfide bond formation, glycosylation, lipidation, phosphorylation, or conjugation with a labeling component.

The term "polynucleotide" refers to a polymeric form of nucleotides of any length, including deoxyribonucleotides or ribonucleotides, or analogs thereof. A polynucleotide may comprise modified nucleotides, such as methylated nucleotides and nucleotide analogs, and may be interrupted by non-nucleotide components. If present, modifications to the nucleotide structure may be imparted before or after assembly of the polymer. The term polynucleotide, as used herein, refers interchangeably to double- and single-stranded molecules. Unless otherwise specified or required, any embodiment of the invention described herein that is a polynucleotide encompasses both the double-stranded form and each of two complementary single-stranded forms known or predicted to make up the double-stranded form.

A polynucleotide or polypeptide has a certain percent "sequence identity" to another polynucleotide or polypeptide, meaning that, when aligned, that percentage of bases or amino acids are the same when comparing the two sequences. Sequence similarity can be determined in a number of different manners. To determine sequence identity, sequences can be aligned using the methods and computer programs, including BLAST, available over the worldwide web at ncbi.nlm.nih.gov/BLAST/. Another alignment algorithm is FASTA, available in the Genetics Computing Group (GCG) package, from Madison, Wis., USA, a wholly owned subsidiary of Oxford Molecular Group, Inc. Other techniques for alignment are described in Methods in Enzymology, vol. 266: Computer Methods for Macromolecular Sequence Analysis (1996), ed. Doolittle, Academic Press, Inc., a division of Harcourt Brace & Co., San Diego, Calif., USA. Of particular interest are alignment programs that permit gaps in the sequence. The Smith-Waterman is one type of algorithm that permits gaps in sequence alignments. See Meth. Mol. Biol. 70: 173-187 (1997). Also, the GAP program using the Needleman and Wunsch alignment method can be utilized to align sequences. See J. Mol. Biol. 48: 443-453 (1970)

Of interest is the BestFit program using the local homology algorithm of Smith and Waterman (Advances in Applied Mathematics 2: 482-489 (1981) to determine sequence identity. The gap generation penalty will generally range from 1 to 5, usually 2 to 4 and in many embodiments will be 3. The gap extension penalty will generally range from about 0.01 to 0.20 and in many instances will be 0.10. The program has default parameters determined by the sequences inputted to be compared. Preferably, the sequence identity is determined using the default parameters determined by the program. This program is available also from Genetics Computing Group (GCG) package, from Madison, Wis., USA.

Another program of interest is the FastDB algorithm. FastDB is described in Current Methods in Sequence Comparison and Analysis, Macromolecule Sequencing and Synthesis, Selected Methods and Applications, pp. 127-149, 1988, Alan R. Liss, Inc. Percent sequence identity is calculated by FastDB based upon the following parameters: Mismatch Penalty: 1.00; Gap Penalty: 1.00; Gap Size Penalty: 0.33; and Joining Penalty: 30.0.

A "promoter" as used herein encompasses a DNA sequence that directs the binding of RNA polymerase and thereby promotes RNA synthesis, i.e., a minimal sequence sufficient to direct transcription. Promoters and corresponding protein or polypeptide expression may be ubiquitous, meaning strongly active in a wide range of cells, tissues and species or cell-type specific, tissue-specific, or species specific. Promoters may be "constitutive," meaning continually active, or "inducible," meaning the promoter can be activated or deactivated by the presence or absence of biotic or abiotic factors. Also included in the nucleic acid constructs or vectors of the invention are enhancer sequences that may or may not be contiguous with the promoter sequence. Enhancer sequences influence promoter-dependent gene expression and may be located in the 5' or 3' regions of the native gene.

"Recombinant," as applied to a polynucleotide means that the polynucleotide is the product of various combinations of cloning, restriction or ligation steps, and other procedures that result in a construct that is distinct from a polynucleotide found in nature.

The terms "treatment", "treating" and the like are used herein to generally mean obtaining a desired pharmacologic and/or physiologic effect. The effect may be prophylactic in terms of completely or partially preventing a disease or symptom thereof, e.g. reducing the likelihood that the disease or symptom thereof occurs in the subject, and/or may be therapeutic in terms of a partial or complete cure for a disease and/or adverse effect attributable to the disease. "Treatment" as used herein covers any treatment of a disease in a mammal, and includes: (a) preventing the disease from occurring in a subject which may be predisposed to the disease but has not yet been diagnosed as having it; (b) inhibiting the disease, i.e., arresting its development; or (c) relieving the disease, i.e., causing regression of the disease. The therapeutic agent may be administered before, during or after the onset of disease or injury. The treatment of ongoing disease, where the treatment stabilizes or reduces the undesirable clinical symptoms of the patient, is of particular interest. Such treatment is desirably performed prior to complete loss of function in the affected tissues. The subject therapy will desirably be administered during the symptomatic stage of the disease, and in some cases after the symptomatic stage of the disease.

As used herein, the phrase "retinal vascular disease" is a disease of the eye, in particular, the retinal caused by aberrant vasculature formation. In some aspects the aberrant vasculature is caused by an inhibition of vasculature development, and in other aspects the aberrant vasculature is cause by excessive angiogenesis.

The practice of the present invention will employ, unless otherwise indicated, conventional techniques of cell biology, molecular biology techniques), microbiology, biochemistry and immunology, which are within the scope of those of skill in the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Handbook of Experimental Immunology" (D. M. Weir & C. C. Blackwell, eds.); "Gene Transfer Vectors for Mammalian Cells" (J. M. Miller & M. P. Calos, eds., 1987); "Current Protocols in Molecular Biology" (F. M. Ausubel et al., eds., 1987); "PCR: The Polymerase Chain Reaction", (Mullis et al., eds., 1994); and "Current Protocols in Immunology" (J. E. Coligan et al., eds., 1991), each of which is expressly incorporated by reference herein.

Several aspects of the invention are described below with reference to example applications for illustration. It should be understood that numerous specific details, relationships, and methods are set forth to provide a full understanding of the invention. One having ordinary skill in the relevant art, however, will readily recognize that the invention can be practiced without one or more of the specific details or with other methods. The present invention is not limited by the illustrated ordering of acts or events, as some acts may occur in different orders and/or concurrently with other acts or events. Furthermore, not all illustrated acts or events are required to implement a methodology in accordance with the present invention.

The terminology used herein is for the purpose of describing particular embodiments only and is not intended to be limiting of the invention. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising".

The term "about" or "approximately" means within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated the term "about" meaning within an acceptable error range for the particular value should be assumed.

All publications mentioned herein are incorporated herein by reference to disclose and describe the methods and/or materials in connection with which the publications are cited. It is understood that the present disclosure supersedes any disclosure of an incorporated publication to the extent there is a contradiction.

It is further noted that the claims may be drafted to exclude any optional element. As such, this statement is intended to serve as antecedent basis for use of such exclusive terminology as "solely", "only" and the like in connection with the recitation of claim elements, or the use of a "negative" limitation.

Unless otherwise indicated, all terms used herein have the same meaning as they would to one skilled in the art and the practice of the present invention will employ, conventional techniques of microbiology and recombinant DNA technology, which are within the knowledge of those of skill of the art.

### II. General.

The present invention provides methods of modulating WNT signals to treat retinopathy, including but limited to, FEVR and other genetic disorders, DR, and AMD. In particular, the present invention provides a WNT/b-catenin agonist and/or antagonist to inhibit aberrant neovascularization in the progression of retinopathy.

WNT ("Wingless-related integration site" or "Wingless and Int-1" or "Wingless-Int") ligands and their signals play key roles in the control of development, homeostasis and regeneration of many essential organs and tissues, including bone, liver, skin, stomach, intestine, kidney, central nervous system, mammary gland, taste bud, ovary, cochlea, lung, and many other tissues (reviewed, e.g., by Clevers, Loh, and Nusse, 2014; 346:1248012). Modulation of WNT signaling pathways has potential for treatment of degenerative diseases and tissue injuries.

One of the challenges for modulating WNT signaling as a therapeutic is the existence of multiple WNT ligands and WNT receptors, Frizzled 1-10 (Fzd1-10), with many tissues expressing multiple and overlapping Fzds. Canonical WNT signals also involve Low-density lipoprotein (LDL) receptor-related protein 5 (LRP5) or Low-density lipoprotein (LDL) receptor-related protein 6 (LRP6) as co-receptors, which are broadly expressed in various tissues, in addition to Fzds.

R-spondins 1-4 are a family of ligands that amplify WNT signals. Each of the R-spondins work through a receptor complex that contains Zinc and Ring Finger 3 (ZNRF3) or Ring Finger Protein 43 (RNF43) on one end and a Leucine-rich repeat-containing G-protein coupled receptor 4-6 (LGR4-6) on the other (reviewed, e.g., by Knight and Hankenson 2014, Matrix Biology; 37: 157-161). R-spondins might also work through additional mechanisms of action. ZNRF3 and RNF43 are two membrane-bound E3 ligases specifically targeting WNT receptors (Fzd1-10 and LRP5 or LRP6) for degradation. Binding of an R-spondin to ZNRF3/RNF43 and LGR4-6 causes clearance or sequestration of the ternary complex, which removes E3 ligases from WNT receptors and stabilizes WNT receptors, resulting in enhanced WNT signals. Each R-spondin contains two Furin domains (1 and 2), with Furin domain 1 binding to ZNRF3/RNF43, and Furin domain 2 binding to LGR4-6. Fragments of R-spondins containing Furin domains 1 and 2 are sufficient for amplifying WNT signaling. While R-spondin effects depend on WNT signals, since both LGR4-6 and ZNRF3/RNF43 are widely expressed in various tissues, the effects of R-spondins are not tissue-specific.

In some embodiments, the WNT/β-catenin signaling antagonist or agonist can include binding agents or epitope binding domains that bind one or more Fzd receptors and inhibit or enhance WNT signaling. In certain embodiments, the agent or antibody specifically binds to the cysteine-rich domain (CRD) within the human frizzled receptor(s) to which it binds. Additionally, antagonistic binding agents containing epitope binding domains against LRP can also be used. In some embodiments, the WNT/β-catenin antagonist possesses binding agents or epitope binding domains that bind E3 ligases ZNRF3/RNF43 and one or more FZD receptors or one or more LRP co-receptors to promote the degradation of FZD or LRP receptors, and this molecule can also contain a binding domain that binds a cell type specific epitope for targeting. The E3 ligase agonist antibodies or fragments thereof can be single molecules or combined with other WNT antagonists, e.g., Fzd receptor antagonists, LRP receptor antagonists, etc.

As is well known in the art, an antibody is an immunoglobulin molecule capable of specific binding to a target such as a carbohydrate, polynucleotide, lipid, polypeptide, etc., through at least on epitope binding domain, located on the variable region of the immunoglobulin molecule. As used herein, the term encompasses not only intact polyclonal or monoclonal antibodies, but also fragments thereof containing epitope binding domains (e.g., dAb, Fab, Fab', (F(ab')₂, Fv, single chain (scFv), VHH or single domain antibodies (sdAb), DVD-Igs, synthetic variants thereof, naturally occurring variants, fusion proteins comprising and epitope binding domain, humanized antibodies, chimeric antibodies, and any other modified configuration of the immunoglobulin molecule that comprises an antigen-binding site or fragment (epitope recognition site) of the required pecificity. "Diabodies," multivalent or multispecific fragments constructed by gene fusion (WO94/13804; P. Holliger et al., Proc. Natl. Acad. Sci. USA 90 6444-6448, 1993) are also a particular form of antibody contemplated herein. Minibodies comprising a scFv joined to a CH3 domain are also included herein (S. Hu et al., Cancer Res., 56, 3055-3061, 1996). See e.g., Ward, E. S. et al., Nature 341, 544-546 (1989); Bird et al., Science, 242, 423-426, 1988; Huston et al., PNAS USA, 85, 5879-5883, 1988); PCT/US92/09965; WO94/13804; P. Holliger et al., Proc. Natl. Acad. Sci. USA 90 6444-6448, 1993; Y. Reiter et al., Nature Biotech, 14, 1239-1245, 1996; S. Hu et al., Cancer Res., 56, 3055-3061, 1996.

The proteolytic enzyme papain preferentially cleaves IgG molecules to yield several fragments, two of which (the F(ab) fragments) each comprise a covalent heterodimer that includes an intact antigen-binding site. The enzyme pepsin is able to cleave IgG molecules to provide several fragments, including the F(ab')2 fragment which comprises both antigen-binding sites. An Fv fragment for use according to certain embodiments of the present disclosure can be produced by preferential proteolytic cleavage of an IgM, and on rare occasions of an IgG or IgA immunoglobulin molecule. Fv fragments are, however, more commonly derived using recombinant techniques known in the art. The Fv fragment includes a non-covalent VH::VL heterodimer including an antigen-binding site which retains much of the antigen recognition and binding capabilities of the native antibody molecule. Inbar et al. (1972) Proc. Nat. Acad. Sci. USA 69:2659-2662; Hochman et al. (1976) Biochem 15:2706-2710; and Ehrlich et al. (1980) Biochem 19:4091-4096.

In certain embodiments, single chain Fv or scFV antibodies are contemplated. For example, Kappa bodies (Ill et al., Prot. Eng. 10: 949-57 (1997)); minibodies (Martin et al., EMBO J 13: 5305-9 (1994)); diabodies (Holliger et al., PNAS 90: 6444-8 (1993)); or Janusins (Traunecker et al., EMBO J 10: 3655-59 (1991) and Traunecker et al., Int. J. Cancer Suppl. 7: 51-52 (1992)), may be prepared using standard molecular biology techniques following the teachings of the present application with regard to selecting antibodies having the desired specificity. In still other embodiments, bispecific or chimeric antibodies may be made that encompass the ligands of the present disclosure. For example, a chimeric antibody may comprise CDRs and framework regions from different antibodies, while bispecific antibodies may be generated that bind specifically to one or more Fzd receptors through one binding domain and to a second molecule through a second binding domain. These antibodies may be produced through recombinant molecular biological techniques or may be physically conjugated together.

A single chain Fv (scFv) polypeptide is a covalently linked VH:VL heterodimer which is expressed from a gene fusion including VH- and VL-encoding genes linked by a peptide-encoding linker. Huston et al. (1988) Proc. Nat. Acad. Sci. USA 85(16):5879-5883. A number of methods have been described to discern chemical structures for converting the naturally aggregated-but chemically separated-light and heavy polypeptide chains from an antibody V region into an scFv molecule which will fold into a three dimensional structure substantially similar to the structure of an antigen-binding site. See, e.g., U.S. Patent Nos. 5,091,513 and 5,132,405, to Huston et al.; and U.S. Patent No. 4,946,778, to Ladner et al.

In certain embodiments, an antibody as described herein is in the form of a diabody. Diabodies are multimers of polypeptides, each polypeptide comprising a first domain comprising a binding region of an immunoglobulin light chain and a second domain comprising a binding region of an immunoglobulin heavy chain, the two domains being linked (e.g., by a peptide linker) but unable to associate with each other to form an antigen binding site: antigen binding sites are formed by the association of the first domain of one polypeptide within the multimer with the second domain of another polypeptide within the multimer (WO94/13804).

A dAb fragment of an antibody consists of a VH domain (Ward, E. S. et al., Nature 341, 544-546 (1989)).

Where bispecific antibodies are to be used, these may be conventional bispecific antibodies, which can be manufactured in a variety of ways (Holliger, P. and Winter G., Current Opinion Biotechnol. 4, 446-449 (1993)), e.g., prepared chemically or from hybrid hybridomas, or may be any of the bispecific antibody fragments mentioned above. Diabodies and scFv can be constructed without an Fc region, using only variable domains, potentially reducing the effects of anti-idiotypic reaction.

Bispecific diabodies, as opposed to bispecific whole antibodies, may also be particularly useful because they can be readily constructed and expressed in E. coli. Diabodies (and many other polypeptides such as antibody fragments) of appropriate binding specificities can be readily selected using phage display (WO94/13804) from libraries. If one arm of the diabody is to be kept constant, for instance, with a specificity directed against antigen X, then a library can be made where the other arm is varied and an antibody of appropriate specificity selected. Bispecific whole antibodies may be made by knobs-into-holes engineering (J. B. B. Ridgeway et al., Protein Eng., 9, 616-621 (1996)).

In certain embodiments, the antibodies described herein may be provided in the form of a UniBody^{®}. A UniBody^{®} is an IgG4 antibody with the hinge region removed (see GenMab Utrecht, The Netherlands; see also, e.g., US20090226421). This proprietary antibody technology creates a stable, smaller antibody format with an anticipated longer therapeutic window than current small antibody formats. IgG4 antibodies are considered inert and thus do not interact with the immune system. Fully human IgG4 antibodies may be modified by eliminating the hinge region of the antibody to obtain half-molecule fragments having distinct stability properties relative to the corresponding intact IgG4 (GenMab, Utrecht). Halving the IgG4 molecule leaves only one area on the UniBody^{®} that can bind to cognate antigens (e.g., disease targets) and the UniBody^{®} therefore binds univalently to only one site on target cells.

In certain embodiments, antibodies and antigen-binding fragments thereof as described herein include a heavy chain and a light chain CDR set, respectively interposed between a heavy chain and a light chain framework region (FR) set which provide support to the CDRs and define the spatial relationship of the CDRs relative to each other. As used herein, the term "CDR set" refers to the three hypervariable regions of a heavy or light chain V region. Proceeding from the N-terminus of a heavy or light chain, these regions are denoted as "CDR1," "CDR2," and "CDR3" respectively. An antigen-binding site, therefore, includes six CDRs, comprising the CDR set from each of a heavy and a light chain V region. A polypeptide comprising a single CDR, (e.g., a CDR1, CDR2 or CDR3) is referred to herein as a "molecular recognition unit." Crystallographic analysis of a number of antigen-antibody complexes has demonstrated that the amino acid residues of CDRs form extensive contact with bound antigen, wherein the most extensive antigen contact is with the heavy chain CDR3. Thus, the molecular recognition units are primarily responsible for the specificity of an antigen-binding site.

As used herein, the term "FR set" refers to the four flanking amino acid sequences which frame the CDRs of a CDR set of a heavy or light chain V region. Some FR residues may contact bound antigen; however, FRs are primarily responsible for folding the V region into the antigen-binding site, particularly the FR residues directly adjacent to the CDRs. Within FRs, certain amino residues and certain structural features are very highly conserved. In this regard, all V region sequences contain an internal disulfide loop of around 90 amino acid residues. When the V regions fold into a binding-site, the CDRs are displayed as projecting loop motifs which form an antigen-binding surface. It is generally recognized that there are conserved structural regions of FRs which influence the folded shape of the CDR loops into certain "canonical" structures-regardless of the precise CDR amino acid sequence. Further, certain FR residues are known to participate in non-covalent interdomain contacts which stabilize the interaction of the antibody heavy and light chains.

A "monoclonal antibody" refers to a homogeneous antibody population wherein the monoclonal antibody is comprised of amino acids (naturally occurring and non-naturally occurring) that are involved in the selective binding of an epitope. Monoclonal antibodies are highly specific, being directed against a single epitope. The term "monoclonal antibody" encompasses not only intact monoclonal antibodies and full-length monoclonal antibodies, but also fragments thereof (such as Fab, Fab', F(ab')2, Fv), single chain (scFv), Nanobodies^{®}, variants thereof, fusion proteins comprising an antigen-binding fragment of a monoclonal antibody, humanized monoclonal antibodies, chimeric monoclonal antibodies, and any other modified configuration of the immunoglobulin molecule that comprises an antigen- binding fragment (epitope recognition site) of the required specificity and the ability to bind to an epitope, including WNT surrogate molecules disclosed herein. It is not intended to be limited as regards the source of the antibody or the manner in which it is made (e.g., by hybridoma, phage selection, recombinant expression, transgenic animals, etc.). The term includes whole immunoglobulins as well as the fragments etc. described above under the definition of "antibody".

In certain embodiments, the antibodies of the present disclosure may take the form of a Nanobody^{®}. Nanobody^{®} technology was originally developed following the discovery and identification that camelidae (e.g., camels and llamas) possess fully functional antibodies that consist of heavy chains only and therefore lack light chains. These heavy-chain only antibodies contain a single variable domain(VHH) and two constant domains (CH2, CH3). The cloned and isolated single variable domains have full antigen binding capacity and are very stable. These single variable domains, with their unique structural and functional properties, form the basis of "Nanobodies^{®}". Nanobodies^{®} are encoded by single genes and are efficiently produced in almost all prokaryotic and eukaryotic hosts, e.g., E. coli (see, e.g., U.S. Pat. No. 6,765,087), molds (for example Aspergillus or Trichoderma) and yeast (for example Saccharomyces, Kluyvermyces, Hansenula or Pichia (see, e.g., U.S. Pat. No. 6,838,254). The production process is scalable and multi-kilogram quantities of Nanobodies^{®} have been produced. Nanobodies^{®} may be formulated as a ready-to-use solution having a long shelf life. The Nanoclone^{®} method (see, e.g., WO 06/079372) is a proprietary method for generating Nanobodies^{®} against a desired target, based on automated high-throughput selection of B-cells. Nanobodies^{®} are single-domain antigen-binding fragments of camelid-specific heavy-chain only antibodies. Nanobodies^{®}, also referred to as VHH antibodies, typically have a small size of around 15 kDa.

Another antibody fragment contemplated is a dual-variable domain-immunoglobulin (DVD-Ig) is an engineered protein that combines the function and specificity of two monoclonal antibodies in one molecular entity. A DVD-Ig is designed as an IgG-like molecule, except that each light chain and heavy chain contains two variable domains in tandem through a short peptide linkage, instead of one variable domain in IgG. The fusion orientation of the two variable domains and the choice of linker sequence are critical to functional activity and efficient expression of the molecule. A DVD-Ig can be produced by conventional mammalian expression systems as a single species for manufacturing and purification. A DVD-Ig has the specificity of the parental antibodies, is stable in vivo, and exhibits IgG-like physicochemical and pharmacokinetic properties. DVD-Igs and methods for making them are described in Wu, C., et al., Nature Biotechnology, 25:1290-1297 (2007)).

In certain embodiments, the antibodies or antigen-binding fragments thereof as disclosed herein are humanized. This refers to a chimeric molecule, generally prepared using recombinant techniques, having an antigen- binding site derived from an immunoglobulin from a non-human species and the remaining immunoglobulin structure of the molecule based upon the structure and/or sequence of a human immunoglobulin. The antigen-binding site may comprise either complete variable domains fused onto constant domains or only the CDRs grafted onto appropriate framework regions in the variable domains. Epitope binding sites may be wild type or modified by one or more amino acid substitutions. This eliminates the constant region as an immunogen in human individuals, but the possibility of an immune response to the foreign variable region remains (LoBuglio, A. F. et al., (1989) Proc Natl Acad Sci USA 86:4220-4224; Queen et al., PNAS (1988) 86:10029-10033; Riechmann et al., Nature (1988) 332:323-327). Illustrative methods for humanization of the anti-Fzd or LRP antibodies disclosed herein include the methods described in U.S. Pat. No. 7,462,697.

Another approach focuses not only on providing human-derived constant regions, but modifying the variable regions as well so as to reshape them as closely as possible to human form. It is known that the variable regions of both heavy and light chains contain three complementarity-determining regions (CDRs) which vary in response to the epitopes in question and determine binding capability, flanked by four framework regions (FRs) which are relatively conserved in a given species and which putatively provide a scaffolding for the CDRs. When nonhuman antibodies are prepared with respect to a particular epitope, the variable regions can be "reshaped" or "humanized" by grafting CDRs derived from nonhuman antibody on the FRs present in the human antibody to be modified. Application of this approach to various antibodies has been reported by Sato, K., et al., (1993) Cancer Res 53:851-856; Riechmann, L., et al., (1988) Nature 332:323-327; Verhoeyen, M., et al., (1988) Science 239:1534-1536; Kettleborough, C. A., et al., (1991) Protein Engineering 4:773-3783; Maeda, H., et al., (1991) Human Antibodies Hybridoma 2:124-134; Gorman, S. D., et al., (1991) Proc Natl Acad Sci USA 88:4181-4185; Tempest, P. R., et al., (1991) Bio/Technology 9:266-271; Co, M. S., et al., (1991) Proc Natl Acad Sci USA 88:2869-2873; Carter, P., et al., (1992) Proc Natl Acad Sci USA 89:4285-4289; and Co, M. S. et al., (1992) J Immunol 148:1149-1154. In some embodiments, humanized antibodies preserve all CDR sequences (for example, a humanized mouse antibody which contains all six CDRs from the mouse antibodies). In other embodiments, humanized antibodies have one or more CDRs (one, two, three, four, five, six) which are altered with respect to the original antibody, which are also termed one or more CDRs "derived from" one or more CDRs from the original antibody.

In certain embodiments, the antibodies of the present disclosure may be chimeric antibodies. In this regard, a chimeric antibody is comprised of an antigen-binding fragment of an antibody operably linked or otherwise fused to a heterologous Fc portion of a different antibody. In certain embodiments, the heterologous Fc domain is of human origin. In other embodiments, the heterologous Fc domain may be from a different Ig class from the parent antibody, including IgA (including subclasses IgA1 and IgA2), IgD, IgE, IgG (including subclasses IgG1, IgG2, IgG3, and IgG4), and IgM. In further embodiments, the heterologous Fc domain may be comprised of CH2 and CH3 domains from one or more of the different Ig classes. As noted above with regard to humanized antibodies, the antigen-binding fragment of a chimeric antibody may comprise only one or more of the CDRs of the antibodies described herein (e.g., 1, 2, 3, 4, 5, or 6 CDRs of the antibodies described herein), or may comprise an entire variable domain (VL, VH or both).

The structures and locations of immunoglobulin CDRs and variable domains may be determined by reference to Kabat, E. A. et al., Sequences of Proteins of Immunological Interest. 4th Edition. US Department of Health and Human Services. 1987, and updates thereof, now available on the Internet (immuno.bme.nwu.edu).

In certain embodiments, the antagonist or agonist binding agent binds with a dissociation constant (K_{D}) of about 1 µM or less, about 100 nM or less, about 40 nM or less, about 20 nM or less, or about 10 nM or less. For example, in certain embodiments, a FZD binding agent or antibody described herein that binds to more than one FZD, binds to those FZDs with a K_{D} of about 100nM or less, about 20 nM or less, or about 10 nM or less. In certain embodiments, the binding agent binds to one or more its target antigen with an EC50 of about 1 µM or less, about 100 nM or less, about 40 nM or less, about 20 nM or less, about 10 nM or less, or about 1 nM 20 or less.

The antibodies or other agents of the present invention can be assayed for specific binding by any method known in the art. The immunoassays which can be used include, but are not limited to, competitive and non-competitive assay systems using techniques such as biolayer interferometry (BLI) analysis, FACS analysis, immunofluorescence, immunocytochemistry, Western blots, radioimmunoassays, ELISA, "sandwich" immunoassays, immunoprecipitation assays, precipitation reactions, gel diffusion precipitin reactions, immunodiffusion assays, agglutination assays, complement-fixation assays, immunoradiometric assays, fluorescent immunoassays, and protein A immunoassays. Such assays are routine and well known in the art (see, e.g., Ausubel et al, eds, 1994, Current Protocols in Molecular Biology, Vol. 1, John Wiley & Sons, Inc., New York, which is incorporated by reference herein in its entirety).

For example, the specific binding of an antibody to a target antigen may be determined using ELISA. An ELISA assay comprises preparing antigen, coating wells of a 96 well microtiter plate with antigen, adding the antibody or other binding agent conjugated to a detectable compound such as an enzymatic substrate (e.g. horse-radish peroxidase or alkaline phosphatase) to the well, incubating for a period of time and detecting the presence of the antigen. In some embodiments, the antibody or agent is not conjugated to a detectable compound, but instead a second conjugated antibody that recognizes the first antibody or agent is added to the well. In some embodiments, instead of coating the well with the antigen, the antibody or agent can be coated to the well and a second antibody conjugated to a detectable compound can be added following the addition of the antigen to the coated well. One of skill in the art would be knowledgeable as to the parameters that can be modified to increase the signal detected as well as other variations of ELISAs known in the art (see e.g. Ausubel et al, eds, 1994, Current Protocols in Molecular Biology, Vol. 1, John Wiley & Sons, Inc., New York at 11.2.1).

The binding affinity of an antibody or other agent to a target antigen and the off-rate of the antibody-antigen interaction can be determined by competitive binding assays. One example of a competitive binding assay is a radioimmunoassay comprising the incubation of labeled antigen (e.g., Fzd, LRP), or fragment or variant thereof, with the antibody of interest in the presence of increasing amounts of unlabeled antigen followed by the detection of the antibody bound to the labeled antigen. The affinity of the antibody and the binding off-rates can be determined from the data by scatchard plot analysis. In some embodiments, BLI analysis is used to determine the binding on and off rates of antibodies or agents. BLI kinetic analysis comprises analyzing the binding and dissociation of antibodies from chips with immobilized antigens on their surface.

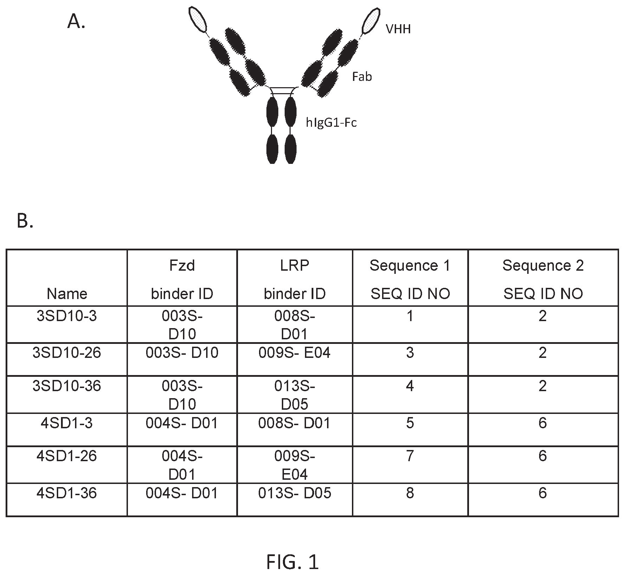

In certain embodiments, the WNT agonist is selected from those disclosed in PCT Publication No. WO2019126398, which is incorporated herein in its entirety. In particular embodiments, a WNT agonist has a structure diagrammed in Fig. 1A and/or comprises the sequences disclosed for any of the WNT agonists disclosed in Fig. 1B. In some embodiments, a WNT agonist comprises a sequence having at least 90% identity (e.g., 95%, 98% or 100% identity) to a sequence disclosed in any of SEQ ID NOs: 1-8, in which the leader sequence is shown in italics, the linker sequence is underlined, and the VHH/sdAb or VH or VL sequence is in bold.

MDMRVPAQLLGLLLLWLRGARCDVQLVESGGGLVQPGGSLRLSCTSSANINSIE
TLGWYRQAPGKQRELIANMRGGGYMKYAGSLKGRFTMSTESAKNTMYLQ
MNSLKPEDTAVYYCYVKLRDDDYVYRGQGTQVTVSSGGSGSDIQMTQSPSSL
SASVGDRVTITCRASQGISSYLAWYQQKPGKAPKLLIYAASNLLGGVPSRFSGSG
SGTDFTLTISSLQPEDFATYYCQQTYSTPWTFGQGTKVEIKRTVAAPSVFIFPPSDE
QLKSGTASVVCLLNNFYPREAKVQWKVDNALQSGNSQESVTEQDSKDSTYSLSS
TLTLSKADYEKHKVYACEVTHQGLSSPVTKSFNRGEC* (SEQ ID NO:1)

MDMRVPAQLLGLLLLWLRGARCEVQLVESGGGLVKPGGSLRLSCAASGFNFGI
YSMTWVRQAPGKGLEWISYISGDSGYTNYADSVKGRFTISRDDSKNTLYLQ
MNSLKTEDTAVYYCARVGPGGWFDPWGQGTLVTVSSASTKGPSVFPLAPSSK
STSGGTAALGCLVKDYFPEPVTVSWNSGALTSGVHTFPAVLQSSGLYSLSSVVTV
PSSSLGTQTYICNVNHKPSNTKVDKKVEPKSCDKTHTCPPCPAPEAAGGPSVFLFP
PKPKDTLMISRTPEVTCVVVDVSHEDPEVKFNWYVDGVEVHNAKTKPREEQYNS
TYRVVSVLTVLHQDWLNGKEYKCKVSNKALGAPIEKTISKAKGQPREPQVYTLP
PSREEMTKNQVSLTCLVKGFYPSDIAVEWESNGQPENNYKTTPPVLDSDGSFFLY
SKLTVDKSRWQQGNVFSCSVMHEALHNHYTQKSLSLSPGK* (SEQ ID NO: 2)

MDMRVPAQLLGLLLLWLRGARCDVQLVESGGGLVQAGGSLRLACAGSGRIFAI
YDIAWYRHPPGNQRELVAMIRPVVTEIDYADSVKGRFTISRNNAMKTVYLQ
MNNLKPEDTAVYYCNAKRPWGSRDEYWGQGTQVTVSSGSGGSDIQMTQSPS
SLSASVGDRVTITCRASQGISSYLAWYQQKPGKAPKLLIYAASNLLGGVPSRFSGS
GSGTDFTLTISSLQPEDFATYYCQQTYSTPWTFGQGTKVEIKRTVAAPSVFIFPPSD
EQLKSGTASVVCLLNNFYPREAKVQWKVDNALQSGNSQESVTEQDSKDSTYSLS
STLTLSKADYEKHKVYACEVTHQGLSSPVTKSFNRGEC* (SEQ ID NO: 3)

MDMRVPAQLLGLLLLWLRGARCQVKLEESGGGLVQAGGSLRLSCAASGRIFSIY
DMGWFRQAPGKEREFVSGIRWSGGTSYADSVKGRFTISKDNAKNTIYLQMN
NLKAEDTAVYYCGSRGYWGQGTLVTVSSGGSGSDIQMTQSPSSLSASVGDRVT
ITCRASQGISSYLAWYQQKPGKAPKLLIYAASNLLGGVPSRFSGSGSGTDFTLTISS

LQPEDFATYYCQQTYSTPWTFGQGTKVEIKRTVAAPSVFIFPPSDEQLKSGTASVV
CLLNNFYPREAKVQWKVDNALQSGNSQESVTEQDSKDSTYSLSSTLTLSKADYE
KHKVYACEVTHQGLSSPVTKSFNRGEC* (SEQ ID NO:4)

MDMRVPAQLLGLLLLWLRGARCDVQLVESGGGLVQPGGSLRLSCTSSANINSIE
TLGWYRQAPGKQRELIANMRGGGYMKYAGSLKGRFTMSTESAKNTMYLQ
MNSLKPEDTAVYYCYVKLRDDDYVVRGQGTQVTVSSGGSGSDIQMTQSPSSL
SASVGDRVTITCRASQSISSYLNWYQQKPGKAPKLLIYAASSLQSGVPSRFSGSGS
GTDFTLTISSLQPEDFATYYCQQSYSTPLTFGGGTKVEIKRTVAAPSVFIFPPSDEQ
LKSGTASVVCLLNNFYPREAKVQWKVDNALQSGNSQESVTEQDSKDSTYSLSST
LTLSKADYEKHKVYACEVTHQGLSSPVTKSFNRGEC* (SEQ ID NO:5)

MDMRVPAQLLGLLLLWLRGARCEVQLVESGGGLVKPGGSLRLSCAASGFTFTN
YAMSWVRQAPGKGLEWVSAISGSGGSTYYADSVKGRFTISRDDSKNTLYLQ
MNSLKTEDTAVYYCARATGFGTVVFDYWGQGTLVTVSSASTKGPSVFPLAPS
SKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPEAAGGPSVF
LFPPKPKDTLMISRTPEVTCVVVDVSHEDPEVKFNWYVDGVEVHNAKTKPREEQ
YNSTYRVVSVLTVLHQDWLNGKEYKCKVSNKALGAPIEKTISKAKGQPREPQVY
TLPPSREEMTKNQVSLTCLVKGFYPSDIAVEWESNGQPENNYKTTPPVLDSDGSF
FLYSKLTVDKSRWQQGNVFSCSVMHEALHNHYTQKSLSLSPGK* (SEQ ID NO:6)

MDMRVPAQLLGLLLLWLRGARCDVQLVESGGGLVQAGGSLRLACAGSGRIFAI
YDIAWYRHPPGNQRELVAMIRPVVTEIDYADSVKGRFTISRNNAMKTVYLQ
MNNLKPEDTAVYYCNAKRPWGSRDEYWGQGTQVTVSSGGSGSDIQMTQSPS
SLSASVGDRVTITCRASQSISSYLNWYQQKPGKAPKLLIYAASSLQSGVPSRFSGS
GSGTDFTLTISSLQPEDFATYYCQQSYSTPLTFGGGTKVEIKRTVAAPSVFIFPPSD
EQLKSGTASVVCLLNNFYPREAKVQWKVDNALQSGNSQESVTEQDSKDSTYSLS
STLTLSKADYEKHKVYACEVTHQGLSSPVTKSFNRGEC* (SEQ ID NO:7)

MDMRVPAQLLGLLLLWLRGARCQVKLEESGGGLVQAGGSLRLSCAASGRIFSIY
DMGWFRQAPGKEREFVSGIRWSGGTSYADSVKGRFTISKDNAKNTIYLQMN
NLKAEDTAVYYCGSRGYWGQGTLVTVSSGGSGSDIQMTQSPSSLSASVGDRVT
ITCRASQSISSYLNWYQQKPGKAPKLLIYAASSLQSGVPSRFSGSGSGTDFTLTISS
LQPEDFATYYCQQSYSTPLTFGGGTKVEIKRTVAAPSVFIFPPSDEQLKSGTASVV
CLLNNFYPREAKVQWKVDNALQSGNSQESVTEQDSKDSTYSLSSTLTLSKADYE
KHKVYACEVTHQGLSSPVTKSFNRGEC* (SEQ ID NO:8)

### III. Pharmaceutical Compositions

Pharmaceutical compositions comprising a WNT antagonist or agonist molecule described herein and one or more pharmaceutically acceptable diluent, carrier, or excipient are also disclosed.

In further embodiments, pharmaceutical compositions comprising a polynucleotide comprising a nucleic acid sequence encoding a WNT antagonist/agonist molecule described herein and one or more pharmaceutically acceptable diluent, carrier, or excipient are also disclosed. In certain embodiments, the polynucleotides are DNA or mRNA, e.g., a modified mRNA. In particular embodiments, the polynucleotides are modified mRNAs further comprising a 5' cap sequence and/or a 3' tailing sequence, e.g., a polyA tail. In other embodiments, the polynucleotides are expression cassettes comprising a promoter operatively linked to the coding sequences.

In some embodiments the WNT antagonist/agonist is an engineered recombinant polypeptide incorporating various epitope binding fragments that bind to various molecules in the WNT signaling pathway. For example, a WNT antagonist can be an antibody or fragment thereof that binds to Fzd4 receptor and/or an LRP receptor and inhibits WNT signaling. The Fzd4 and LRP antibody fragments (e.g., Fab, scFv, VHH/sdAbs, etc.) may be joined together directly or with various size linkers, on one molecule.

Conversely, engineered WNT agonists/antagonists can also be recombinant polypeptides incorporating epitope binding fragments that bind to various molecules in the WNT signaling pathway and enhance WNT signaling. For example, a WNT agonist can be an antibody or fragment thereof that binds to Fzd receptor and/or an LRP receptor and enhances WNT signaling. The Fzd and LRP antibody fragments (e.g., Fab, scFv, VHH/sdAbs, etc.) may be joined together directly or with various size linkers, on one molecule.

In further embodiments, pharmaceutical compositions comprising an expression vector, e.g., a viral vector, comprising a polynucleotide comprising a nucleic acid sequence encoding a WNT antagonist/agonist molecule described herein and one or more pharmaceutically acceptable diluent, carrier, or excipient are also disclosed. In certain embodiments, the nucleic acid sequence encoding the WNT antagonist molecule and the nucleic acid sequence encoding the WNT agonist are in the same polynucleotide, e.g., expression cassette.

The present disclosure further contemplates a pharmaceutical composition comprising a cell comprising an expression vector comprising a polynucleotide comprising a promoter operatively linked to a nucleic acid encoding a WNT antagonist/agonist molecule and one or more pharmaceutically acceptable diluent, carrier, or excipient. In particular embodiments, the pharmaceutical composition further comprises a cell comprising an expression vector comprising a polynucleotide comprising a promoter operatively linked to a nucleic acid sequence encoding a WNT antagonist and a WNT agonist. In certain embodiments, the nucleic acid sequence encoding the WNT antagonist molecule and the nucleic acid sequence encoding the WNT agonist molecule are present in the same polynucleotide, e.g., expression cassette and/or in the same cell. In particular embodiments, the cell is a heterologous cell or an autologous cell obtained from the subject to be treated.

In particular embodiments, the cell is a stem cell, e.g., an adipose-derived stem cell or a hematopoietic stem cell. The present disclosure contemplates pharmaceutical compositions comprising a first molecule for delivery of a WNT antagonist molecule as a first active agent, and a WNT agonist as a second molecule. The first and second molecule may be the same type of molecule or different types of molecules. For example, in certain embodiments, the first and second molecule may each be independently selected from the following types of molecules: polypeptides, small organic molecules, nucleic acids encoding the first or second active agent (optionally DNA or mRNA, optionally modified RNA), vectors comprising a nucleic acid sequence encoding the first or second active agent (optionally expression vectors or viral vectors), and cells comprising a nucleic acid sequence encoding the first or second active agent (optionally an expression cassette).

The subject molecules, alone or in combination, can be combined with pharmaceuticallyacceptable carriers, diluents, excipients and reagents useful in preparing a formulation that is generally safe, non-toxic, and desirable, and includes excipients that are acceptable for mammalian, e.g., human or primate, use. Such excipients can be solid, liquid, semisolid, or, in the case of an aerosol composition, gaseous. Examples of such carriers, diluents and excipients include, but are not limited to, water, saline, Ringer's solutions, dextrose solution, and 5% human serum albumin. Supplementary active compounds can also be incorporated into the formulations. Solutions or suspensions used for the formulations can include a sterile diluent such as water for injection, saline solution, fixed oils, polyethylene glycols, glycerine, propylene glycol or other synthetic solvents; antibacterial compounds such as benzyl alcohol or methyl parabens; antioxidants such as ascorbic acid or sodium bisulfite; chelating compounds such as ethylenediaminetetraacetic acid (EDTA); buffers such as acetates, citrates or phosphates; detergents such as Tween 20 to prevent aggregation; and compounds for the adjustment of tonicity such as sodium chloride or dextrose. The pH can be adjusted with acids or bases, such as hydrochloric acid or sodium hydroxide. In particular embodiments, the pharmaceutical compositions are sterile.

Pharmaceutical compositions may further include sterile aqueous solutions or dispersions and sterile powders for the extemporaneous preparation of sterile injectable solutions or dispersion. For intravenous administration, suitable carriers include physiological saline, bacteriostatic water, or phosphate buffered saline (PBS). In some cases, the composition is sterile and should be fluid such that it can be drawn into a syringe or delivered to a subject from a syringe. In certain embodiments, it is stable under the conditions of manufacture and storage and is preserved against the contaminating action of microorganisms such as bacteria and fungi. The carrier can be, e.g., a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the internal compositions can be brought about by including in the composition an agent which delays absorption, for example, aluminum monostearate and gelatin.

Sterile solutions can be prepared by incorporating the WNT antagonist/agonist antibody or antigen-binding fragment thereof (or encoding polynucleotide or cell comprising the same) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile- filtered solution thereof.

In one embodiment, the pharmaceutical compositions are prepared with carriers that will protect the antibody or antigen-binding fragment thereof against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially. Liposomal suspensions can also be used as pharmaceutically acceptable carriers. These can be prepared according to methods known to those skilled in the art.

It may be advantageous to formulate the pharmaceutical compositions in dosage unit form for ease of administration and uniformity of dosage. Dosage unit form as used herein refers to physically discrete units suited as unitary dosages for the subject to be treated; each unit containing a predetermined quantity of active antibody or antigen-binding fragment thereof calculated to produce the desired therapeutic effect in association with the required pharmaceutical carrier. The specification for the dosage unit forms are dictated by and directly dependent on the unique characteristics of the antibody or antigen-binding fragment thereof and the particular therapeutic effect to be achieved, and the limitations inherent in the art of compounding such an active antibody or antigen-binding fragment thereof for the treatment of individuals.

The pharmaceutical compositions can be included in a container, pack, or dispenser, e.g. syringe, e.g. a prefilled syringe, together with instructions for administration.

The pharmaceutical compositions of the present disclosure encompass any pharmaceutically acceptable salts, esters, or salts of such esters, or any other compound which, upon administration to an animal comprising a human, is capable of providing (directly or indirectly) the biologically active antibody or antigen-binding fragment thereof.

The present disclosure includes pharmaceutically acceptable salts of a WNT antagonist/agonist molecule described herein. The term "pharmaceutically acceptable salt" refers to physiologically and pharmaceutically acceptable salts of the compounds of the present disclosure: i.e., salts that retain the desired biological activity of the parent compound and do not impart undesired toxicological effects thereto. A variety of pharmaceutically acceptable salts are known in the art and described, e.g., in "Remington's Pharmaceutical Sciences", 17th edition, Alfonso R. Gennaro (Ed.), Mark Publishing Company, Easton, PA, USA, 1985 (and more recent editions thereof), in the "Encyclopaedia of Pharmaceutical Technology", 3rd edition, James Swarbrick (Ed.), Informa Healthcare USA (Inc.), NY, USA, 2007, and in J. Pharm. Sci. 66:2 (1977). Also, for a review on suitable salts, see "Handbook of Pharmaceutical Salts: Properties, Selection, and Use" by Stahl and Wermuth (Wiley-VCH, 2002). Pharmaceutically acceptable base addition salts are formed with metals or amines, such as alkali and alkaline earth metals or organic amines.

Metals used as cations comprise sodium, potassium, magnesium, calcium, and the like. Amines comprise N-N'-dibenzylethylenediamine, chloroprocaine, choline, diethanolamine, dicyclohexylamine, ethylenediamine, N- methylglucamine, and procaine (see, for example, Berge et al., "Pharmaceutical Salts," J. Pharma Sci., 1977, 66, 119). The base addition salts of said acidic compounds are prepared by contacting the free acid form with a sufficient amount of the desired base to produce the salt in the conventional manner. The free acid form may be regenerated by contacting the salt form with an acid and isolating the free acid in the conventional manner. The free acid forms differ from their respective salt forms somewhat in certain physical properties such as solubility in polar solvents, but otherwise the salts are equivalent to their respective free acid for purposes of the present disclosure.

In some embodiments, the pharmaceutical composition provided herein comprise a therapeutically effective amount of a WNT antagonist/agonist molecule or pharmaceutically acceptable salt thereof in admixture with a pharmaceutically acceptable carrier, diluent and/or excipient, for example saline, phosphate buffered saline, phosphate and amino acids, polymers, polyols, sugar, buffers, preservatives and other proteins. Exemplary amino acids, polymers and sugars and the like are octylphenoxy polyethoxy ethanol compounds, polyethylene glycol monostearate compounds, polyoxyethylene sorbitan fatty acid esters, sucrose, fructose, dextrose, maltose, glucose, mannitol, dextran, sorbitol, inositol, galactitol, xylitol, lactose, trehalose, bovine or human serum albumin, citrate, acetate, Ringer's and Hank's solutions, cysteine, arginine, carnitine, alanine, glycine, lysine, valine, leucine, polyvinylpyrrolidone, polyethylene and glycol. Preferably, this formulation is stable for at least six months at 4° C.

In some embodiments, the pharmaceutical composition provided herein comprises a buffer, such as phosphate buffered saline (PBS) or sodium phosphate/sodium sulfate, tris buffer, glycine buffer, sterile water and other buffers known to the ordinarily skilled artisan such as those described by Good et al. (1966) Biochemistry 5:467. The pH of the buffer may be in the range of 6.5 to 7.75, preferably 7 to 7.5, and most preferably 7.2 to 7.4.

### IV. Methods of Use

The present disclosure also provides methods for using the WNT antagonist/agonist molecules, e.g., to modulate a WNT signaling pathway, e.g., to increase or decrease WNT signaling, and the administration of a WNT antagonist/agonist molecule in a variety of therapeutic settings. Provided herein are methods of treatment using a WNT antagonist/agonist molecule. In one embodiment, a WNT antagonist/agonist molecule is provided to a subject having a disease involving inappropriate or deregulated WNT signaling.

In certain embodiments, a WNT antagonist/agonist molecule may be used to block or enhance a WNT signaling pathway in a tissue or a cell. Antagonizing the WNT signaling pathway may include decreasing or inhibiting WNT signaling in a cell or tissue. Agonizing the WNT signaling pathway may include, for example, increasing WNT signaling or enhancing WNT signaling in a tissue or cell. Thus, in some aspects, the present disclosure provides a method for antagonizing/agonizing a WNT signaling pathway in a cell, comprising contacting the tissue or cell with an effective amount of a WNT antagonist/agonist molecule or pharmaceutically acceptable salt thereof disclosed herein, wherein the WNT antagonist/agonist molecule is a WNT signaling pathway antagonist/agonist. In some embodiments, contacting occurs in vitro, ex vivo, or in vivo. In particular embodiments, the cell is a cultured cell, and the contacting occurs in vitro.

The WNT antagonist/agonist molecule may be used for the treatment of retinopathy. In particular, activation of WNT signaling is necessary for retinal vascularization during vessel development in eye. Genetic deletion of norrin, Fzd4, Lrp5, or Tspan12 significantly regresses not only vascular development on superficial retina surface, but also vascular penetration into deeper layers of retina. Additionally, the generated avascular area due to immature vascularization causes ischemia-induced neovascularization. Therefore, the timely controlled administrations of WNT agonist or/and antagonist not only will regress retinopathy disease progression but also would also lead to an improvement of the illness. In the particular embodiments, WNT agonist/antagonist will be administered in either earlier or later phase of retinopathy disease progression in the subjects.

Both WNT agonist and antagonist may be administered alone as a monotherapy or sequentially. Administration of agonist in earlier phase of disease development, which shows avascular area in retina, would stimulate/stabilize vessel formation and protect the vessels from avascular factors. On the other hand, administration of antagonist in later phase which shows neovascularization could inhibit the aberrant vessel regeneration in retina. Therefore, the sequential treatment of both agonist and antagonist is one potential option to modulate the disease. In a representative dosing schedule, agonist is administered first in avascularization phase and then followed by application of antagonist in neovascularization phase. For testing the opposing roles, the WNT agonist and antagonist will be administered in reverse sequence order into subjects. However, given the potential effects of WNT on stabilization of vessel structure, administration of agonist in the neovascularization phase is also considered.

Retinal vascular diseases can include, but are not limited to: familiar exudative vitreoretionopathy (FEVR), exudative vitreoretinopathy, Norrie disease, diabetic retinopathy (DR), age-related macular degeneration (AMD), retinopathy of prematurity (ROP), osteoporosis-psuedoglioma syndrome (OPPG), retinal vein occlusion, and Coats disease.

The present invention also provides for combination treatment with known treatments for FEVR and/or DR. For example, the WNT antagonist/agonist can be administered in combination with current therapy for retinopathy, including, but not limited to, anti-VEGF antibody. In some embodiments, anti-Ang2 antibody will also be administered to subjects in combination with WNT agonist/antagonist. Hypoxia-induced VEGF and Ang2 expression are important cues for pathological neovascularization, and indeed, an antagonist Ang2 antibody has been considered for retinopathy patient treatment (Gadkar et al., Invest Ophthalmol Vis Sci. 2015 Aug;56(9):5390-400). The anti-VEGF antibody or anti-Ang2 antibody can be administered sequentially or concurrently with the molecules of the present invention. VEGF antagonists can include, but are not limited to: bevacizumab, ranibizumab, aflibercept, ramucirumab, and tanibirumab, and Ang2 antagonists can include but are not limited to: nesvacumab, AMG780, and MEDI3617.

In a further embodiment, the antagonist and/or agonist molecule may also incorporate a tissue targeting moiety, e.g., an antibody or fragment thereof that recognizes a retinal tissue specific receptor or cell surface molecule.

The therapeutic agent (e.g., a WNT antagonist/agonist) may be administered before, during or after the onset of disease or injury. The treatment of ongoing disease, where the treatment stabilizes or reduces the undesirable clinical symptoms of the patient, is of particular interest. Such treatment is desirably performed prior to complete loss of function in the affected tissues. The subject therapy will desirably be administered during the symptomatic stage of the disease, and in some cases after the symptomatic stage of the disease. In some embodiments, the subject method results in a therapeutic benefit, e.g., preventing the development of a disorder, halting the progression of a disorder, reversing the progression of a disorder, etc. In some embodiments, the subject method comprises the step of detecting that a therapeutic benefit has been achieved. The ordinarily skilled artisan will appreciate that such measures of therapeutic efficacy will be applicable to the particular disease being modified, and will recognize the appropriate detection methods to use to measure therapeutic efficacy.

All of the above U.S. patents, U.S. patent application publications, U.S. patent applications, foreign patents, foreign patent applications and non- patent publications referred to in this specification and/or listed in the Application Data Sheet, are incorporated herein by reference, in their entirety.

From the foregoing it will be appreciated that, although specific embodiments of the present disclosure have been described herein for purposes of illustration, various modifications may be made without deviating from the spirit and scope of the present disclosure. Accordingly, the present disclosure is not limited except as by the appended claims.

The broad scope of this invention is best understood with reference to the following example, which is not intended to limit the inventions to the specific embodiments.

### EXAMPLE 1

### I. General methods

Standard methods in molecular biology are described. Maniatis et al. (1982) Molecular Cloning, A Laboratory Manual, Cold Spring Harbor Laboratory Press, Cold Spring Harbor, N.Y.; Sambrook and Russell (2001) Molecular Cloning, 3rd ed., Cold Spring Harbor Laboratory Press, Cold Spring Harbor, N.Y.; Wu (1993) Recombinant DNA, Vol. 217, Academic Press, San Diego, Calif. Standard methods also appear in Ausbel et al. (2001) Current Protocols in Molecular Biology, Vols. 1-4, John Wiley and Sons, Inc. New York, N.Y., which describes cloning in bacterial cells and DNA mutagenesis (Vol. 1), cloning in mammalian cells and yeast (Vol. 2), glycoconjugates and protein expression (Vol. 3), and bioinformatics (Vol. 4).

Methods for protein purification including immunoprecipitation, chromatography, electrophoresis, centrifugation, and crystallization are described. Coligan et al. (2000) Current Protocols in Protein Science, Vol. 1, John Wiley and Sons, Inc., New York. Chemical analysis, chemical modification, post-translational modification, production of fusion proteins, glycosylation of proteins are described. See, e.g., Coligan et al. (2000) Current Protocols in Protein Science, Vol. 2, John Wiley and Sons, Inc., New York; Ausubel et al. (2001) Current Protocols in Molecular Biology, Vol. 3, John Wiley and Sons, Inc., NY, N.Y., pp. 16.0.5-16.22.17; Sigma-Aldrich, Co. (2001) Products for Life Science Research, St. Louis, Mo.; pp. 45-89; Amersham Pharmacia Biotech (2001) BioDirectory, Piscataway, N.J., pp. 384-391. Production, purification, and fragmentation of polyclonal and monoclonal antibodies are described. Coligan et al. (2001) Current Protocols in Immunology, Vol. 1, John Wiley and Sons, Inc., New York; Harlow and Lane (1999) Using Antibodies, Cold Spring Harbor Laboratory Press, Cold Spring Harbor, N.Y.; Harlow and Lane, supra. Standard techniques for characterizing ligand/receptor interactions are available. See, e.g., Coligan et al. (2001) Current Protocols in Immunology, Vol. 4, John Wiley, Inc., New York.

Methods for flow cytometry, including fluorescence activated cell sorting detection systems (FACS^{®}), are available. See, e.g., Owens et al. (1994) Flow Cytometry Principles for Clinical Laboratory Practice, John Wiley and Sons, Hoboken, N.J.; Givan (2001) Flow Cytometry, 2nd ed.; Wiley-Liss, Hoboken, N.J.; Shapiro (2003) Practical Flow Cytometry, John Wiley and Sons, Hoboken, N.J. Fluorescent reagents suitable for modifying nucleic acids, including nucleic acid primers and probes, polypeptides, and antibodies, for use, e.g., as diagnostic reagents, are available. Molecular Probes (2003) Catalogue, Molecular Probes, Inc., Eugene, Oreg.; Sigma-Aldrich (2003) Catalogue, St. Louis, Mo.

Standard methods of histology of the immune system are described. See, e.g., Muller-Harmelink (ed.) (1986) Human Thymus: Histopathology and Pathology, Springer Verlag, New York, N.Y.; Hiatt, et al. (2000) Color Atlas of Histology, Lippincott, Williams, and Wilkins, Phila, Pa.; Louis, et al. (2002) Basic Histology: Text and Atlas, McGraw-Hill, New York, N.Y.

Software packages and databases for determining, e.g., antigenic fragments, leader sequences, protein folding, functional domains, glycosylation sites, and sequence alignments, are available. See, e.g., GenBank, Vector NTI^{®} Suite (Informax, Inc, Bethesda, Md.); GCG Wisconsin Package (Accelrys, Inc., San Diego, Calif.); DeCypher^{®} (TimeLogic Corp., Crystal Bay, Nev.); Menne et al. (2000) Bioinformatics 16: 741-742; Menne et al. (2000) Bioinformatics Applications Note 16:741-742; Wren et al. (2002) Comput. Methods Programs Biomed. 68:177-181; von Heijne (1983) Eur. J. Biochem. 133:17-21; von Heijne (1986) Nucleic Acids Res. 14:4683-4690.

### II. FZD4 WNT Surrogates

Monospecific FZD4 WNT surrogates (3SD10-3, 3SD10-26, 3SD10-36, 4SD1-3, 4SD1-26, and 4SD1-36) were constructed as described in PCT Publication No. WO2019126398. Fig. 1A provides a graphical representation of the structure of the WNT surrogate molecules used, and Fig. 1B is a table indicating the Fzd binding domain and LRP binding domain present in the WNT surrogates, and providing the sequences present in the indicated WNT surrogates. Specificity for the FZD4 receptor was tested as described below.

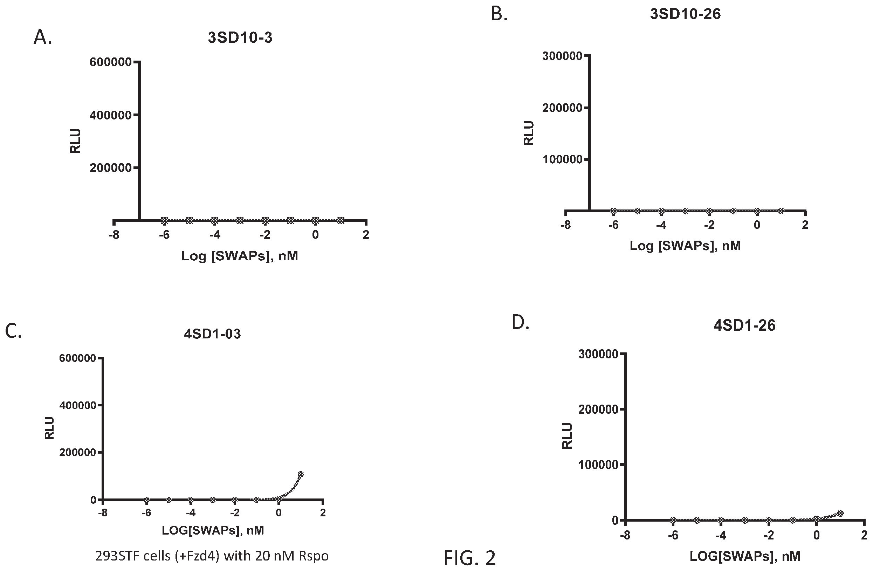

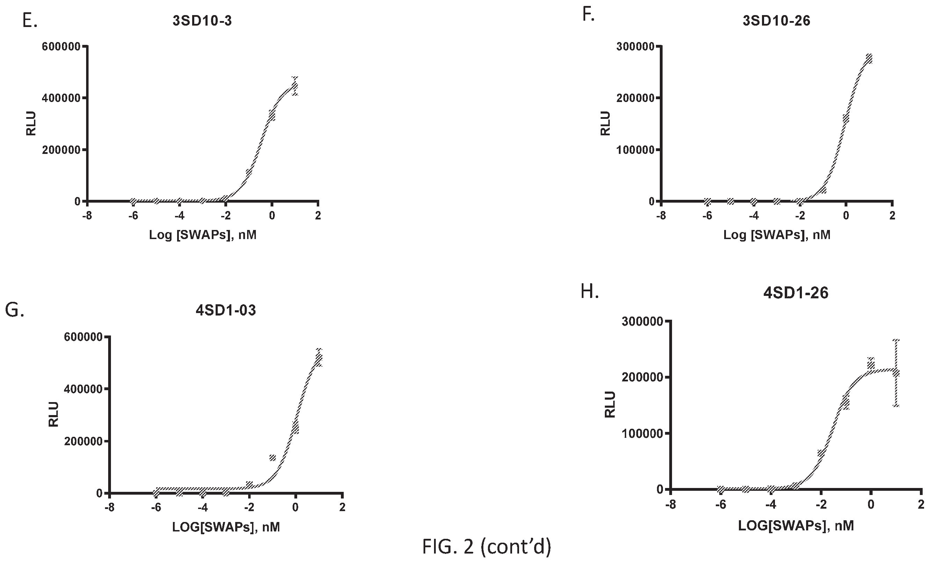

WNT signaling activity was measured using a HEK293 cell line (293STF) containing a luciferase gene controlled by a WNT-responsive promoter (293STF) as previously reported (see, e.g., Janda et al. (2017) Nature 545:234-237). In brief, the 293STF cells were seeded at a density of 10,000 per well in 96-well plates 24 hr prior to treatment, then treated by 3SD10-3, 3SD10-26, 4SD1-3, or 4SD1-26, together with 20 nM of Rspo. Cells were lysed with Luciferase Cell Culture Lysis Reagent (Promega) and activity was measured with Luciferase Assay System (Promega) using vendor suggested procedures. Data were plotted as average -/+ standard deviation of triplicates and fitted by non-linear regression using Prism (GraphPad Software). For over expression of FZD4, cells were transiently transfected with plasmid containing human FZD4 gene under CMV promoter (OHu21807 from GenScript), then split into 96-well plates for STF assay 24 hours post transfection. Figs. 2A-2D shows no WNT signaling activity in untransfected 293STF cells. In contrast, cells transiently transfected with FZD4 receptor exhibited WNT signaling (Figs. 2E-2H).

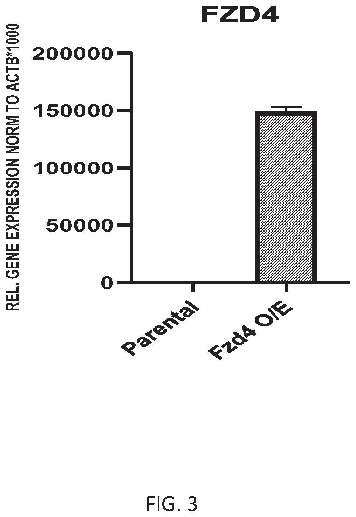

RNA from parental or FZD4 overexpressed 293STF cells was extracted using the Qiagen RNeasy Micro Kit (Qiagen, 74004). cDNA was produced using the SuperScript^{™} VILO^{™} cDNA Synthesis Kit (ThermoFisher, 11754050). human FZD4 expression was measured by using TaqMan^{®} Fast Advanced Master Mix (ThermoFisher, 4444963) and the Hs00201853_m1 FZD4 probe (ThermoFisher, 4331182). Values were normalized to expression of constitutive ACTIN B gene using the Hs01060665_m1 probe (ThermoFisher, 4331182). Fig. 3 illustrates gene expression levels of FZD4 transiently transfected cells over expressing FZD4.

### III. WNT Activity in Additional Cell Lines

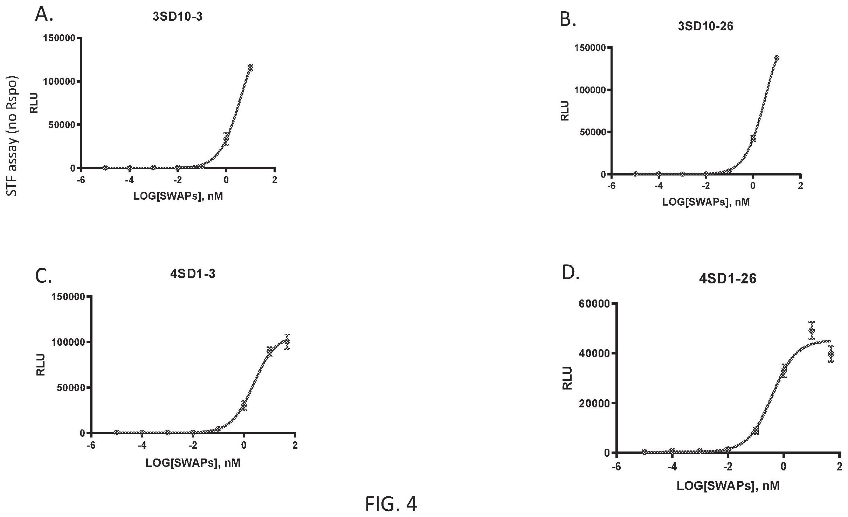

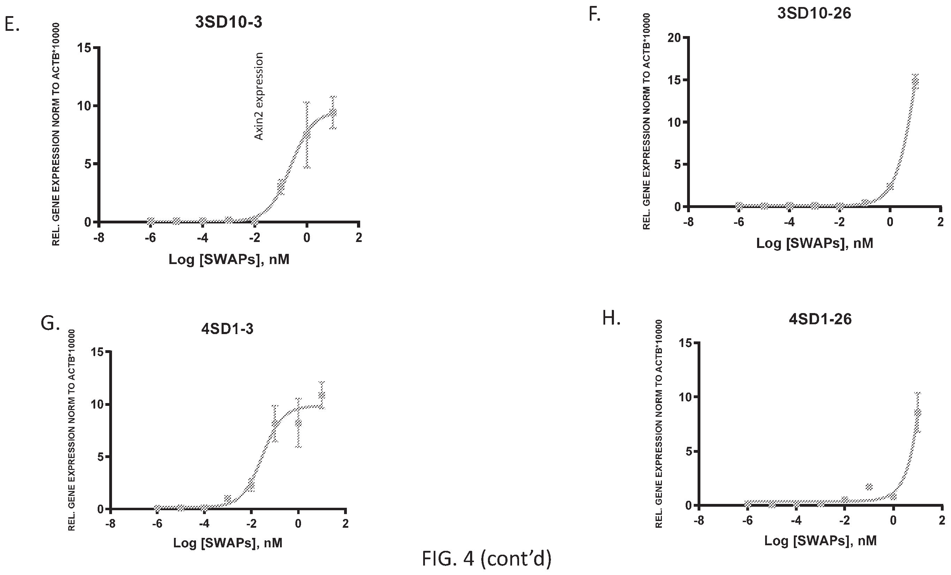

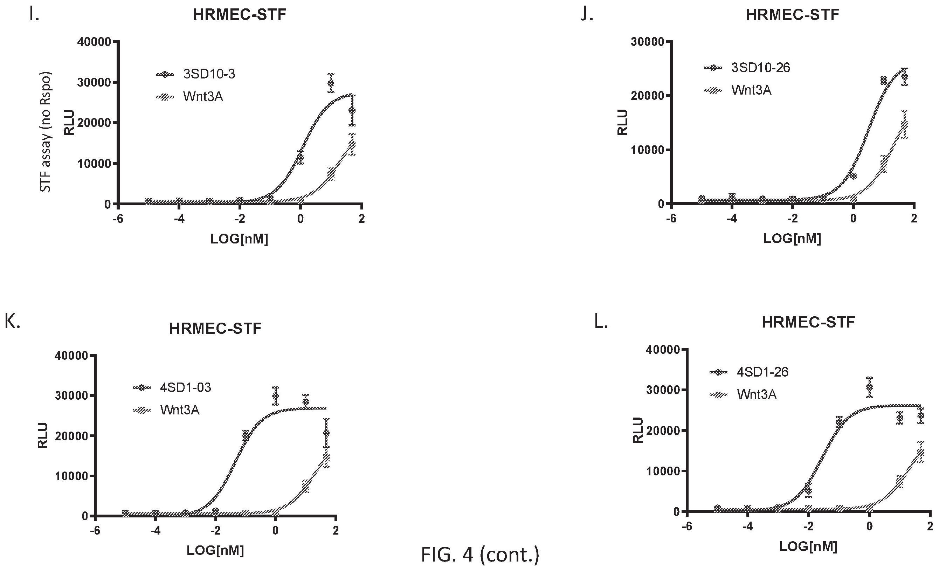

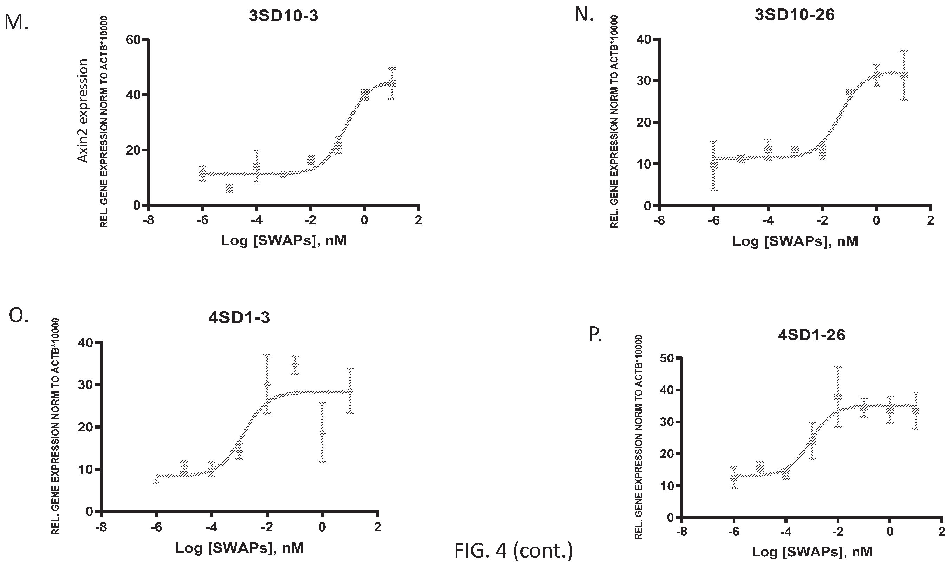

WNT signaling activity was measured using bEnd.3 (mouse brain endothelial cell line used in vascular studies) or HRMEC (Primary Human Retinal Microvascular Endothelial Cells) cells containing a luciferase gene controlled by a WNT-responsive promoter. Cells were transiently transfected with STF plasmid encoding the firefly luciferase reporter under the control of a minimal promoter and a concatemer of seven LEF/TCF binding sites. The transfected cells were seeded at a density of 10,000 per well in 96-well plates 24 hours prior to treatment, then treated by 3SD10-3, 3SD10-26, 4SD1-3, 4SD1-26 or WNT3a. Cells were lysed with Luciferase Cell Culture Lysis Reagent (Promega) and activity was measured with Luciferase Assay System (Promega) using vendor suggested procedures. Data were plotted as average -/+ standard deviation of triplicates and fitted by non-linear regression using Prism (GraphPad Software). Figures 4A-4H shows increased WNT signaling activity and Axin2 expression in bEnd.3 cells treated with the monoFZD4 WNT surrogate. Figures 4I-4P showed similar WNT signaling and Axin2 expression increases in the HRMEC cells.

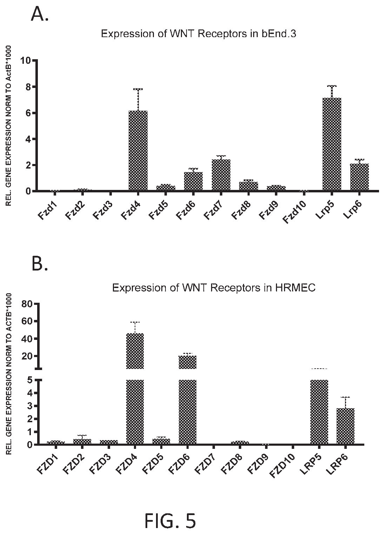

RNA from bEnd.3 and HRMEC cells was extracted using the Qiagen RNeasy Micro Kit (Qiagen, 74004). cDNA was produced using the SuperScript^{™} VILO^{™} cDNA Synthesis Kit (ThermoFisher, 11754050). The indicated human gene expressions in HRMEC were measured by using TaqMan^{®} Fast Advanced Master Mix (ThermoFisher, 4444963) and the Hs00268943_s1 FZD1, Hs00361432_s1 FZD2, Hs00184043_m1 FZD3, Hs00201853_m1 FZD4, Hs00258278_s1 FZD5, Hs00171574_m1 FZD6, Hs00275833_s1 FZD7, Hs00259040_s1 FZD8, Hs00268954_s1 FZD9, Hs00273077_s1 FZD10, Hs00182031_m1 LRP5, Hs00233945_m1 LRP6, Hs00610344_m1 AXIN2 probes (ThermoFisher, 4331182). Values were normalized to expression of constitutive ACTIN B gene using the Hs01060665_m1 probe (ThermoFisher, 4331182). The indicated mouse gene expressions in bEnd.3 cells were measured by using TaqMan^{®} Fast Advanced Master Mix (ThermoFisher, 4444963) and the Mm00445405_s1 Fzd1, Mm02524776_s1 Fzd2, Mm00445423_m1 Fzd3, Mm00433382_m1 Fzd4, Mm00445623_s1 Fzd5, Mm00433387_m1 Fzd6, Mm00433409_s1 Fzd7, Mm01234717_s1 Fzd8, Mm01206511_s1 Fzd9, Mm00558396_s1 Fzd10, Mm01227476_m1 Lrp5, Mm00999795_m1 Lrp6, Mm00443610_m1 Axin2 probes (ThermoFisher, 4331182). Values were normalized to expression of constitutive Actin B gene using the Mm02619580_g1 probe (ThermoFisher, 4331182). Data for Axin2 expression were plotted as average -/+ standard deviation of triplicates and fitted by non-linear regression using Prism (GraphPad Software). Figs. 5A and 5B show expression of WNT receptors in bEnd.3 cells and HRMEC, respectively.

### IV. Effect of RSPO on FZD4 WNT Surrogate Activity

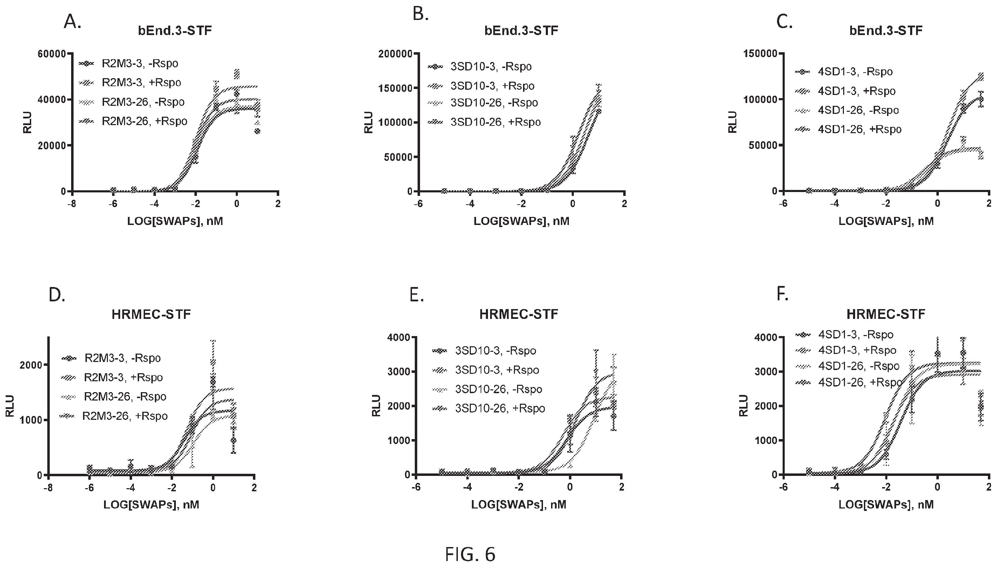

WNT signaling activity was measured using bEnd.3 or HRMEC cells containing a luciferase gene controlled by a WNT-responsive promoter. Cells were transiently transfected with STF plasmid encoding the firefly luciferase reporter under the control of a minimal promoter and a concatemer of seven LEF/TCF binding sites. The transfected cells were seeded at a density of 10,000 per well in 96-well plates 24 hr prior to treatment, then treated by R2M3-3, R2M3-26, 3SD10-3, 3SD10-26, 4SD1-3, 4SD1-26 (see, e.g., WO2019126398) together with or without 20 nM Rspo. Cells were lysed with Luciferase Cell Culture Lysis Reagent (Promega) and activity was measured with Luciferase Assay System (Promega) using vendor suggested procedures. Data were plotted as average - /+ standard deviation of triplicates and fitted by non-linear regression using Prism (GraphPad Software). Figs. 6A-6F shows that addition of RSPO with the different FZD4 WNT surrogates in both types of endothelial cells had little significant effect on WNT signaling activity.

### V. Oxygen-induced Retinopathy

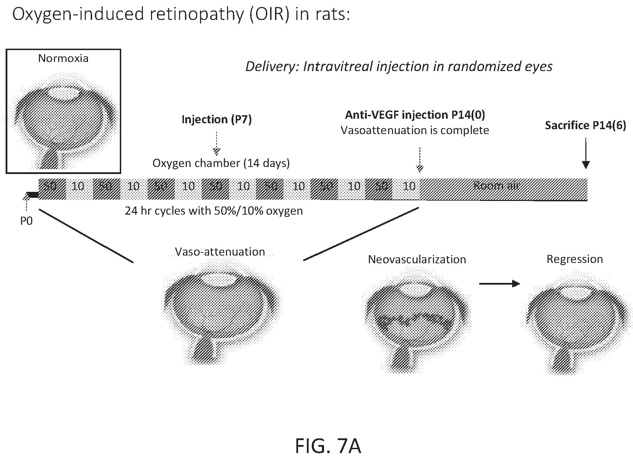

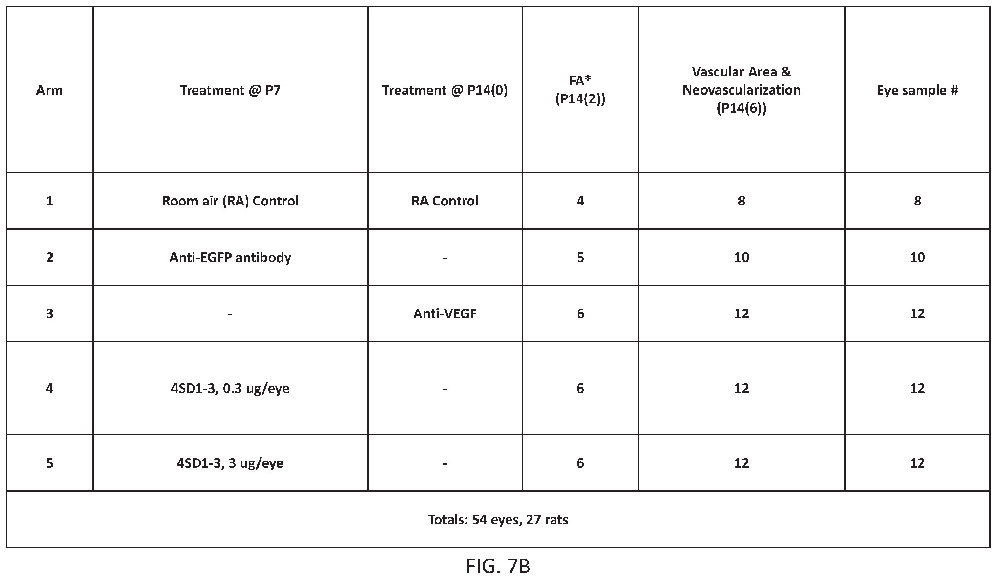

Within 8 hours of birth, litters of Sprague-Dawley rat pups and their mothers were transferred to oxygen exposure chambers in which they were subjected to alternating 24-hour periods of 50% and 10% oxygen for 14 days (i.e., P1-P14). On postnatal day 14, or P14(0), the oxygen-exposed rats were returned to room air. They remained in room air for an additional six days, P14(1) through P14(6). Age matched rat litters also were maintained in room air (RA) to serve as controls. Each eye of the rats in three arms received an intravitreal injection of 3 ug anti-EGFP Ab, 0.3 ug 4SD1-03, or 3 ug 4SD1-03 at P7, while those in another arm received an intravitreal injection of anti-VEGF treatment at P14(0) (see, e.g., study design depicted in Figs. 7A and 7B).

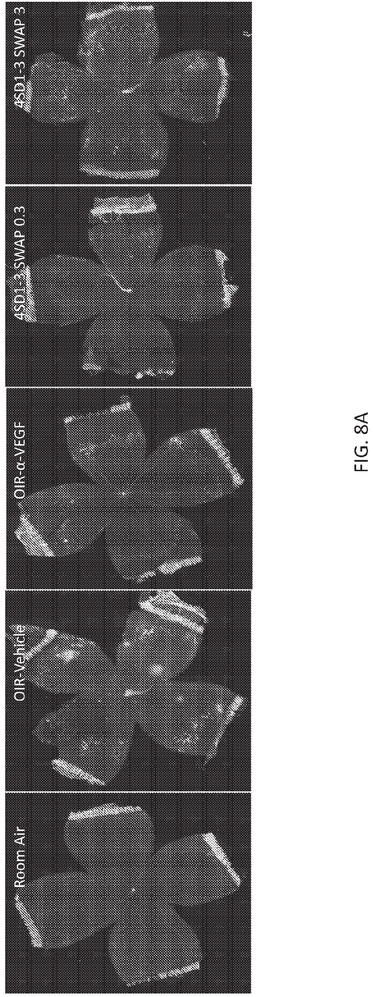

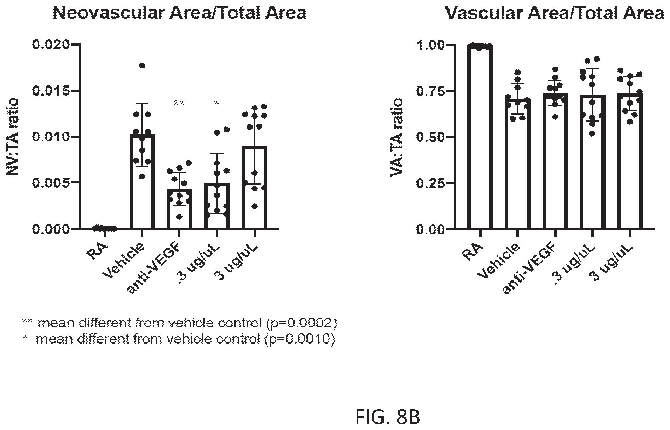

Following treatments, all rats were sacrificed on P14(6), at which time both normal intra-retinal vascular growth and pathological pre-retinal neovascularization (NV) were assessed in isolectin-B4-stained retinal flatmounts, using computer-assisted image analysis of high-resolution digital images. TA: total area. Figs. 8A-8B show 0.3 µg of 4SD1-3 inhibited neovascular tuft formation to a similar extent as anti-VEGF treatment. This demonstrates that FZD4 WNT surrogate treatment has comparable effects to anti-VEGF treatment in this model of retinopathy.

The various embodiments described above can be combined to provide further embodiments. All of the U.S. patents, U.S. patent application publications, U.S. patent applications, foreign patents, foreign patent applications and non-patent publications referred to in this specification and/or listed in the Application Data Sheet are incorporated herein by reference, in their entirety. Aspects of the embodiments can be modified, if necessary to employ concepts of the various patents, applications and publications to provide yet further embodiments. These and other changes can be made to the embodiments in light of the above-detailed description.

In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

### EMBODIMENTS

Various embodiments of the invention are defined in the clauses below:
1. A method of treating a retinopathy in a subject, comprising administering an engineered WNT signaling modulator to the subject.
2. The method of clause 1, wherein the WNT signaling modulator is an engineered WNT agonist or an engineered WNT antagonist.
3. The method of clause 2, wherein the engineered WNT agonist and engineered WNT antagonist comprise binding compositions that bind to one or more Fzd receptors and binding compositions that bind to one or more LRP receptors or Tspan 12 receptors.
4. The method of clause 3, wherein the binding compositions of the engineered WNT agonist are selected from the group consisting of a Fzd4 binding composition, a Lrp5 binding composition, a Lrp6 binding composition, a LRP5/6 binding composition, and a Tspan12 binding composition.
5. The method of clause 1, comprising administering an engineered WNT agonist and an engineered WNT antagonist, wherein the engineered WNT agonist and engineered WNT antagonist are administered independently at early and/or late stages of the retinopathy.
6. The method of clause 1, comprising administering an engineered WNT agonist and an engineered WNT antagonist, wherein the engineered WNT agonist and the engineered WNT antagonist are administered sequentially at early and/or late stages of the retinopathy.
7. The method of clause 1, comprising administering an engineered WNT agonist and an engineered WNT antagonist, wherein the engineered WNT agonist and the engineered WNT antagonist are co-administered at early and/or late stages of the retinopathy.
8. The method of clause 6, wherein the WNT agonist is administered before or after the WNT antagonist.
9. The method of any of clause 1-7, comprising administering an engineered WNT agonist and an engineered WNT antagonist, wherein the WNT agonist and/or the WNT antagonist is administered with a binding composition specific for either VEGF and/or Ang2.
10. The method of clause 9, wherein the binding composition specific for VEGF or Ang2 is an antagonist of VEGF or Ang2 activity.
11. The method of clause 10, wherein the VEGF antagonist is selected from the group consisting of: bevacizumab, ranibizumab, aflibercept, ramucirumab, and tanibirumab.
12. The method of clause 10, wherein the Ang2 antagonist is selected from the group consisting of nesvacumab, AMG780, and MEDI3617.
13. The method of any one of clause 1-12, wherein the retinopathy is a retinal vascular disease.
14. The method of clause 13, wherein the retinal vascular disease is caused by inhibition of vascular development.
15. The method of clause 13, wherein the retinopathy is caused by excessive angiogenesis.
16. The method of clause 13 or clause 14, wherein the retinal vascular disease is selected from the group consisting of: familiar exudative vitreoretionopathy (FEVR), exudative vitreoretinopathy, Norrie disease, diabetic retinopathy (DR), age-related macular degeneration (AMD), retinopathy of prematurity (ROP), osteoporosis-psuedoglioma syndrome (OPPG), retinal vein occlusion, and Coats disease.

## Claims

1. An engineered WNT signaling modulator for use in a method of treating a retinopathy in a subject, the method comprising administering the engineered WNT signaling modulator to the subject, wherein the engineered WNT signaling modulator comprises a sequence having at least 90% identity to SEQ **ID** NO: 5 and a sequence having at least 90% identity to SEQ **ID** NO: 6.

2. The engineered WNT signaling modulator for the use of claim 1, wherein the WNT signaling modulator is an engineered WNT agonist.

3. The engineered WNT signaling modulator for the use of claim 2, wherein the engineered WNT agonist comprises binding compositions that bind to one or more Fzd receptors and binding compositions that bind to one or more LRP receptors.

4. The engineered WNT signaling modulator for the use of claim 3, wherein the engineered WNT signaling modulator is an engineered WNT agonist comprising binding compositions selected from the group consisting of a Fzd4 binding composition, a Lrp5 binding composition, a Lrp6 binding composition, a LRP5/6 binding composition.

5. The engineered WNT signaling modulator for the use of claim 1, the method comprising administering an engineered WNT agonist and an engineered WNT antagonist, wherein the engineered WNT agonist and engineered WNT antagonist are administered independently or sequentially at early and/or late stages of the retinopathy.

6. The engineered WNT signaling modulator for the use of claim 1, the method comprising administering an engineered WNT agonist and an engineered WNT antagonist, wherein the engineered WNT agonist and the engineered WNT antagonist are co-administered at early and/or late stages of the retinopathy.

7. The engineered WNT signaling modulator for the use of claim 5, wherein the WNT agonist is administered before or after the WNT antagonist.

8. The engineered WNT signaling modulator for the use of any of claims 1-6, the method comprising administering an engineered WNT agonist and an engineered WNT antagonist, wherein the WNT agonist and/or the WNT antagonist is administered with a binding composition specific for either VEGF and/or Ang2.

9. The engineered WNT signaling modulator for the use of claim 8, wherein the binding composition specific for VEGF or Ang2 is an antagonist of VEGF or Ang2 activity.

10. The engineered WNT signaling modulator for the use of claim 9, wherein the VEGF antagonist is selected from the group consisting of: bevacizumab, ranibizumab, aflibercept, ramucirumab, and tanibirumab.

11. The engineered WNT signaling modulator for the use of claim 9, wherein the Ang2 antagonist is selected from the group consisting of nesvacumab, AMG780, and MEDI3617.

12. The engineered WNT signaling modulator for the use of any one of claims 1-11, wherein the retinopathy is a retinal vascular disease.

13. The engineered WNT signaling modulator for the use of claim 12, wherein the retinal vascular disease is caused by inhibition of vascular development or wherein the retinopathy is caused by excessive angiogenesis.

14. The engineered WNT signaling modulator for the use of claim 12 or claim 13, wherein the retinal vascular disease is selected from the group consisting of: familiar exudative vitreoretionopathy (FEVR), exudative vitreoretinopathy, Norrie disease, diabetic retinopathy (DR), age-related macular degeneration (AMD), retinopathy of prematurity (ROP), osteoporosis-psuedoglioma syndrome (OPPG), retinal vein occlusion, and Coats disease.
